# FASCICULE DE BREVET EUROPEEN

(11) **EP 2 297 576 B1**
(45) Date de publication et mention de la délivrance du brevet: **11.01.2017**
(21) Numéro de dépôt: 09769262.8
(22) Date de dépôt: 23.06.2009
(51) Int. Cl.: C12N 5/0775, C12N 5/077, G01N 33/50

(54) **LIGNEE ETABLIE D'ADIPOCYTES BRUNS HUMAINS ET PROCEDE DE DIFFERENCIATION A PARTIR D'UNE LIGNEE DE CELLULES HMADS**
ETABLIERTE MENSCHLICHE ZELLLINIE VON BRAUNEM FETTGEWEBE UND VERFAHREN ZUR DIFFERENZIERUNG AUS EINER HMADS-ZELLLINIE
ESTABLISHED HUMAN BROWN ADIPOCYTE LINE AND METHOD FOR DIFFERENTIATION FROM AN HMADS CELL LINE

(30) Priorité: 23.06.2008 FR 0854140
(43) Date de publication de la demande: 23.03.2011
(73) Titulaire: Centre National de la Recherche Scientifique (C.N.R.S.), 75016 Paris (FR); UNIVERSITE DE NICE SOPHIA-ANTIPOLIS, 06000 Nice (FR)
(72) Inventeur: AILHAUD, Gérard, Paul, F-83590 Gonfaron (FR); AMRI, Ez-Zoubir, F-06100 Nice (FR); DANI, Christian, Jean, Lucien, F-06100 Nice (FR); ELABD, Christian, F-06100 Nice (FR)
(74) Mandataire: Regimbeau
(86) Numéro de dépôt international: PCT/EP2009/057852
(87) Numéro de publication internationale: WO 2009/156413

(56) Documents cités:
- WO-A2-96/33724
- TIRABY CLAIRE ET AL: "Acquirement of brown fat cell features by human white adipocytes." JOURNAL OF BIOLOGICAL CHEMISTRY, vol. 278, no. 35, 29 août 2003 (2003-08-29), pages 33370-33376, XP002505383 ISSN: 0021-9258 cité dans la demande
- ZILBERFARB V ET AL: "Human immortalized brown adipocytes express functional beta3-adrenoceptor coupled to lipolysis." JOURNAL OF CELL SCIENCE APR 1997, vol. 110 ( Pt 7), avril 1997 (1997-04), pages 801-807, XP002505376 ISSN: 0021-9533 cité dans la demande
- FARMER S R: "Molecular determinants of brown adipocyte formation and function" GENES AND DEVELOPMENT 20080515 US, vol. 22, no. 10, 15 mai 2008 (2008-05-15), pages 1269-1275, XP002505377 ISSN: 0890-9369 1549-5477
- NEDERGAARD J ET AL: "Unexpected evidence for active brown adipose tissue in adult humans" AMERICAN JOURNAL OF PHYSIOLOGY - ENDOCRINOLOGY AND METABOLISM 200708 US, vol. 293, no. 2, août 2007 (2007-08), pages E444-E452, XP002505378 ISSN: 0193-1849 1522-1555 cité dans la demande
- RODRIGUEZ A-M ET AL: "The human adipose tissue is a source of multipotent stem cells" BIOCHIMIE, MASSON, PARIS, FR, vol. 87, no. 1, 1 janvier 2005 (2005-01-01), pages 125-128, XP004768702 ISSN: 0300-9084 cité dans la demande
- RODRIGUEZ A-M ET AL: "Adipocyte differentiation of multipotent cells established from human adipose tissue" BIOCHEMICAL AND BIOPHYSICAL RESEARCH COMMUNICATIONS, ACADEMIC PRESS INC. ORLANDO, FL, US, vol. 315, no. 2, 5 mars 2004 (2004-03-05), pages 255-263, XP004487272 ISSN: 0006-291X cité dans la demande
- TIRABY CLAIRE ET AL: "Conversion from white to brown adipocytes: A strategy for the control of fat mass?" TRENDS IN ENDOCRINOLOGY AND METABOLISM, vol. 14, no. 10, décembre 2003 (2003-12), pages 439-441, XP002505379 ISSN: 1043-2760 cité dans la demande
- WILSON-FRITCH LEANNE ET AL: "Mitochondrial remodeling in adipose tissue associated with obesity and treatment with rosiglitazone" JOURNAL OF CLINICAL INVESTIGATION, vol. 114, no. 9, novembre 2004 (2004-11), pages 1281-1289, XP002505380 ISSN: 0021-9738 cité dans la demande
- WILSON-FRITCH LEANNE ET AL: "Mitochondrial biogenesis and remodeling during adipogenesis and in response to the insulin sensitizer rosiglitazone." MOLECULAR AND CELLULAR BIOLOGY, vol. 23, no. 3, février 2003 (2003-02), pages 1085-1094, XP002505381 ISSN: 0270-7306 cité dans la demande
- KANG SONA ET AL: "Effects of Wnt signaling on brown adipocyte differentiation and metabolism mediated by PGC-1alpha" MOLECULAR AND CELLULAR BIOLOGY, vol. 25, no. 4, février 2005 (2005-02), pages 1272-1282, XP002505382 ISSN: 0270-7306
- SEALE PATRICK ET AL: "Transcriptional control of brown fat determination by PRDM16" CELL METABOLISM, vol. 6, no. 1, juillet 2007 (2007-07), pages 38-54 URL, XP002505384 ISSN: 1550-4131 cité dans la demande
- KREY G ET AL: 'FATTY ACIDS, EICOSANOIDS, AND HYPOLIPIDEMIC AGENTS IDENTIFIED AS LIGANDS OF PEROXISOME PROLIFERATOR-ACTIVATED RECEPTORS BY COACTIVATOR-DEPENDENT RECEPTOR LIGAND ASSAY' CONFERENCE PROCEEDINGS vol. 11, no. 6, 01 Juin 1997, pages 779 - 791, XP002402069
- BUCKLE D R ET AL: "Non thiazolidinedione antihyperglycaemic agents. 1: alpha-heteroatom substituted beta-phenylpropanoic acids", BIOORGANIC & MEDICINAL CHEMISTRY LETTERS, PERGAMON, ELSEVIER SCIENCE, GB, vol. 6, no. 17, 3 September 1996 (1996-09-03), pages 2121-2126, XP004135669, ISSN: 0960-894X, DOI: 10.1016/0960-894X(96)00383-6
- CARPÉNÉ C: "Assays of adrenergic receptors. Including lipolysis and binding measurements.", METHODS IN MOLECULAR BIOLOGY (CLIFTON, N.J.) 2001, vol. 155, 2001, pages 129-140, XP009164817, ISSN: 1064-3745
- RODRIGUEZ A-M ET AL: "Transplantation of a multipotent cell population from human adipose tissue induces dystrophin expression in the immunocompetent mdx mouse", THE JOURNAL OF EXPERIMENTAL MEDICINE, ROCKEFELLER UNIVERSITY PRESS, US, vol. 201, no. 9, 2 May 2005 (2005-05-02), pages 1397-1405, XP003005684, ISSN: 0022-1007, DOI: 10.1084/JEM.20042224
- Laure-Emmanuelle Zaragosi ET AL: "Autocrine Fibroblast Growth Factor 2 Signaling Is Critical for Self-Renewal of Human Multipotent Adipose-Derived Stem Cells", Stem Cells, vol. 24, no. 11, 1 November 2006 (2006-11-01), pages 2412-2419, XP055108785, ISSN: 1066-5099, DOI: 10.1634/stemcells.2006-0006
- ELABD C ET AL: "Human adipose tissue-derived multipotent stem cells differentiate in vitro and in vivo into osteocyte-like cells", BIOCHEMICAL AND BIOPHYSICAL RESEARCH COMMUNICATIONS, ACADEMIC PRESS INC. ORLANDO, FL, US, vol. 361, no. 2, 21 September 2007 (2007-09-21), pages 342-348, XP027016318, ISSN: 0006-291X, DOI: 10.1016/J.BBRC.2007.06.180 [retrieved on 2007-08-06]

## Description

L'invention a pour objet un procédé de différenciation de cellules hMADS en une population d'adipocytes bruns humains fonctionnelle, un procédé de conversion d'une population d'adipocytes blancs humains en une population d'adipocytes bruns humains fonctionnelle, ainsi qu'une méthode de criblage de molécules susceptibles de moduler le poids corporel chez un sujet.

Le tissu adipeux blanc (WAT) joue chez l'homme un rôle-clef dans le contrôle du bilan énergétique et de l'homéostasie glucido-lipidique (Ailhaud G. et al, Annu Rev Nutr, 1992. 12: p. 207-233 ; Rosen, E.D. et B.M. Spiegelman, Nature, 2006. 444(7121): p. 847-53). A l'opposé du WAT, le tissu adipeux brun (BAT) est spécialisé dans la thermogénèse adaptative au cours de laquelle la protéine découplante UCP1 joue un rôle déterminant. La présence de tissu adipeux brun est bien connue chez les rongeurs comme chez les mammifères nouveaux-nés de plus grande taille (Garruti, G. et D. Ricquier, Int J Obes Relat Metab Disord, 1992. 16(5): p. 383-90 ; Cannon, B. et J. Nedergaard, Physiol Rev, 2004. 84(1): p. 277-359). Fait particulièrement frappant, des données récentes permettent d'avancer l'existence de BAT fonctionnel chez l'homme adulte sain (Nedergaard, J. et al, Am J Physiol Endocrinol Metab, 2007. 293(2): p. E444-52; Cypess, AM et al, N. Engl. J. Med. 2009. 360 : p. 1509-17 ; Saito, M. et al, Diabetes 2009. Publish Ahead of Print, Online April 28; van Marken Lichtenbelt, W et al, N. Engl. J. Med. 2009. 390: p. 1500-08; Virtanen, KA et al, N. Engl. J. Med. 2009. 360 : p. 1518-1525).

*In vivo*, l'apparition chez les rongeurs d'îlots d'adipocytes bruns au milieu des dépôts de WAT est connue (Timmons, J.A., et al., Proc Natl Acad Sci USA, 2007. 104(11): p. 4401-6 ; Cousin, B., et al., J Cell Sci, 1992. 103 (Pt 4): p. 931-42 ; Xue, B., et al., Mol Cell Biol, 2005. 25(18): p. 8311-22), à la suite d'une exposition au froid ou d'un traitement par les agonistes des récepteurs β-adrénergiques (β-AR). De même, après un traitement par des ligands agonistes de PPARγ, l'apparition de cellules exprimant UCP1 dans des dépôts adipeux blancs a également été documentée chez les rongeurs et chez l'homme (Wilson-Fritch, L., et al., J Clin Invest, 2004. 114(9): p. 1281-9 ; Fukui, Y., et al., Diabetes, 2000. 49(5): p. 759-67 ; Bogacka, I., et al., Diabetes, 2005. 54(5): p. 1392-9). Toutefois, ces observations ne peuvent exclure l'existence préalable de précurseurs bruns dans de tels sites. Réciproquement, la conversion rapide du BAT en WAT chez le nouveau-né humain comme chez les nouveaux-nés ovins et bovins ne peut exclure l'existence préalable de précurseurs blancs (Casteilla, L., et al., Am J Physiol, 1987. 252(5 Pt 1): p. E627-36 ; Casteilla, L., et al., Biochem J, 1989. 257(3): p. 665-71 ; Casteilla, L., et al., Eur J Biochem, 1991. 198(1): p. 195-9). L'existence d'une cellule précurseur commune aux lignages blanc et brun reste à démontrer, dans la mesure où les préadipocytes obtenus *ex-vivo* à partir du WAT et du BAT ne donnent respectivement naissance qu'à des adipocytes blancs et bruns (Moulin, K., et al., Biochem J, 2001. 356(Pt 2): p. 659-64). Des travaux récents chez la souris favorisent cette possibilité en démontrant l'existence d'une signature transcriptomique myogénique des adipocytes bruns distincte de celle des adipocytes blancs (Timmons, J.A., et al., Proc Natl Acad Sci USA, 2007. 104(11): p. 4401-6).

Toutefois, d'autres travaux soulignent la possibilité de générer des adipocytes bruns à partir d'adipocytes blancs par transgénèse (Tiraby, C. et D. Langin, Trends Endocrinol Metab, 2003. 14(10): p. 439-41 ; Tiraby, C., et al., J Biol Chem, 2003. 278(35): p. 33370-6) et plusieurs co-activateurs et facteurs de transcription participent à la formation d'adipocytes bruns. Ainsi, PGC-1α et PGC-1β jouent au cours de la différenciation un rôle essentiel et complémentaire dans la mitochondriogénèse et la respiration (Puigserver, P., et al., Mol Cell, 2001. 8(5): p. 971-82 ; Uldry, M., et al., Cell Metab, 2006. 3(5): p. 333-41). Cependant, contrairement à ces co-activateurs de PPARγ, la protéine transcriptionnelle en doigt de zinc PRDM16 contrôle véritablement la détermination « brune » des préadipocytes blancs par induction de PGC-1α, d'UCP1 et de la triiodothyronine desiodinase de type 2 (Dio2) (Seale, P., et al., Cell Metab, 2007. 6(1): p. 38-54).

L'article Zilberfarb et al., 1997, J Cell Sci 110 (Pt 7), 801-807, décrit une lignée immortalisée d'adipocytes bruns humains, PAZ6, qui est obtenue par transgénèse. Cet article montre que la lignée PAZ6 exprime l'ARNm codant UCP1.

Toutefois, il ne montre pas que cette expression conduit à une protéine UCP1 fonctionnelle, c'est-à-dire qui possède une activité découplante en raison de sa localisation dans la membrane interne mitochondriale, conférant ainsi aux adipocytes bruns humains une activité respiratoire et une activité découplante significativement supérieures à celles des adipocytes blancs humains. En outre, aucune stimulation de l'activité respiratoire par un agoniste spécifique des récepteurs β-adrénergiques n'est rapportée comme étant dépendante de la présence d'UCP1.

De plus, l'article Zilberfarb et al., 1997 décrit que le récepteur β3-adrénergique est couplé à l'adénylate cyclase et à la lipolyse dans les cellules PAZ6. Ce couplage entre le récepteur β3-adrénergique et l'adénylate cyclase est connu comme étant le préliminaire d'une cascade d'événements conduisant à l'activité découplante de la protéine UCP1.

Or l'article Zilberfarb et al., 1997 décrit également que des résultats similaires de couplage entre l'adénylate cyclase et le récepteur β3-adrénergique ont été obtenus par d'autres auteurs (Murphy et al., 1993) en utilisant des adipocytes de rat fraîchement isolés. Il s'agit en fait d'adipocytes blancs (cf. Titre de Murphy et al., 1993 : «Corrélation of β3-adrenoreceptor-induced activation of cyclic AMP dependent protein kinase with activation of lipolysis in rat white adipocytes»).

Ainsi, les résultats obtenus et décrits dans Zilberfarb et al., 1997 avec les adipocytes bruns PAZ6 sont similaires à ceux obtenus avec des adipocytes blancs, ce qui conduit inévitablement à une activité respiratoire et à une activité découplante de ces adipocytes bruns similaires à celles d'adipocytes blancs. Les adipocytes bruns PAZ6 décrits dans Zilberfarb et al., 1997 ne sont donc pas fonctionnels au sens de la présente invention.

Récemment nous avons isolé à partir de tissu adipeux humain des cellules souches mésenchymateuses (cellules hMADS). Ces cellules présentent à l'état clonal un caryotype normal, une forte capacité d'auto-renouvellement, et une absence de tumorigénicité. Les cellules hMADS se révèlent être capables de se différencier en plusieurs lignages, en particulier ostéoblastique comme adipocytaire, et conduisent *in vivo* à une régénération osseuse et musculaire (Rodriguez, A.M., et al., Biochem Biophys Res Commun, 2004. 315(2): p. 255-63 ; Rodriguez, A.M., et al., J Exp Med, 2005. 201(9): p. 1397-405 ; Zaragosi, L.E., et al, Stem Cells, 2006. 24(11): p. 2412-9 ; Elabd, C., et al., Biochem Biophys Res Commun, 2007. 361(2): p. 342-8). Une fois différenciées en adipocytes, les cellules hMADS acquièrent les propriétés fonctionnelles des adipocytes blancs humains (sécrétion de leptine et d'adiponectine, réponses à l'insuline et aux agonistes β-adrénergiques et, spécifique des primates, au facteur atrial natriurétique, Rodriguez, A.M., et al., Biochem Biophys Res Commun, 2004. 315(2): p. 255-63). Les cellules hMADS représentent donc un modèle cellulaire approprié pour étudier leur capacité éventuelle à se différencier également en adipocytes bruns.

Nos résultats montrent qu'une activation chronique prolongée de PPARγ suffit à promouvoir leur conversion *in vitro* et à augmenter leurs capacités respiratoire et découplante, et que les agonistes des récepteurs β-adrénergiques, y compris les agonistes β3, modulent positivement l'expression d'UCP1 ainsi que la stimulation de l'activité respiratoire par un agoniste spécifique des récepteurs β-adrénergiques tel que l'isoprotérénol. La différenciation des cellules hMADS aboutit à une population d'adipocytes bruns humains fonctionnelle qui peut être utilisée en tant que modèle cellulaire, notamment pour identifier des molécules susceptibles de moduler le poids corporel, et en particulier pour traiter le surpoids et/ou l'obésité.

Ainsi, la présente invention décrit une population d'adipocytes bruns humains fonctionnelle qui exprime simultanément les gènes suivants :
- les gènes codant les protéines UCP1, CIDEA, CPT1B, Bcl2, l'expression de ces gènes étant supérieure à celle d'une population d'adipocytes blancs humains,
- le gène codant la protéine Bax, l'expression de ce gène étant inférieure à celle d'une population d'adipocytes blancs humains, et
- les gènes codant les protéines PPAR alpha, PGC1 alpha, PGC1 bêta et PRDM16, l'expression de ces gènes étant similaire à celle d'une population d'adipocytes blancs humains.

La population (ou lignée) d'adipocytes bruns humains fonctionnelle décrite ici est issue d'une population (ou lignée) de cellules souches humaines multipotentes dérivées du tissu adipeux (cellules hMADS). Les cellules hMADS sont décrites notamment dans la demande internationale publiée le 12 février 2004 sous le numéro WO 2004/013275 et dans Rodriguez, A.M., et al., Biochem Biophys Res Commun, 2004. 315(2): p. 255-63. Ces cellules hMADS sont capables de se différencier en divers types cellulaires tels que des adipocytes, des ostéoblastes, des myocytes et des cellules endothéliales en fonction du milieu de culture utilisé. Les populations établies de cellules hMADS présentent une capacité d'autorenouvellement et de différenciation adipocytaire conservées pendant un certain nombre de doublements de population, c'est-à-dire qu'elles sont viables à long terme et qu'elles peuvent se diviser sans altérer les caractéristiques cellulaires initiales. On parle donc également de lignées de cellules hMADS. Avantageusement, les populations cellulaires hMADS selon la présente invention présentent une capacité d'autorenouvellement et une capacité de différenciation adipocytaire conservées pendant au moins 130, encore plus avantageusement au moins 200 doublements de population. Dans la présente invention, les conditions permettant la différenciation de la population cellulaire hMADS en une population d'adipocytes bruns humains fonctionnelle ont été déterminées et sont décrites ci-après. Une population d'adipocytes bruns humains fonctionnelle a ainsi pu être établie. Celle-ci possède la même capacité d'autorenouvellement et de différenciation adipocytaire conservées pendant un certain nombre de doublements de population que la population cellulaire hMADS dont elle est issue. Elle est donc viable à long terme et peut se diviser sans altérer les caractéristiques cellulaires initiales. On peut donc parler également de lignée d'adipocytes bruns fonctionnelle.

Par population d'adipocytes bruns humains « fonctionnelle » selon la présente invention on entend désigner une population possédant une activité respiratoire et une activité découplante significativement supérieures par rapport à l'activité respiratoire et l'activité découplante de tout autre type cellulaire, notamment par rapport à celles des adipocytes blancs. En outre, l'activité respiratoire significativement supérieure de la population d'adipocytes bruns humains fonctionnelle est stimulée lorsque cette population est exposée à un agoniste spécifique des récepteurs β-adrénergiques tel que l'isoprotérénol. L'activité respiratoire et l'activité découplante significativement supérieures dans la population d'adipocytes bruns humains fonctionnelle, ainsi que la stimulation de l'activité respiratoire par un agoniste spécifique des récepteurs β-adrénergiques, sont dues à l'expression du gène codant UCP1 par rapport à l'absence d'expression du même gène dans des adipocytes blancs humains. L'activité découplante de la protéine UCP1 résulte de sa présence dans la membrane interne mitochondriale des adipocytes bruns humains. Une activité respiratoire et une activité découplante significativement supérieures de ces adipocytes bruns humains ne seraient pas observées si la protéine UCP1 n'était pas à la fois localisée et fonctionnelle dans la membrane interne mitochondriale. La fonctionnalité des adipocytes bruns humains peut être vérifiée de différentes manières, décrites ci-après.

Dans la présente invention, on entend par « activité respiratoire » la capacité d'une cellule ou d'une population cellulaire à consommer de l'oxygène. Cette capacité à consommer de l'oxygène peut être mesurée comme indiqué ci-après.

Dans la présente invention, on entend par « activité découplante » la capacité de la protéine UCP1, et par conséquent la capacité de la cellule qui la contient, à découpler son activité respiratoire de sa production d'ATP. En effet, lorsque la cellule respire, elle produit un gradient de protons entre l'intérieur et l'extérieur de la membrane interne mitochondriale. Ce gradient permet la génération d'ATP. Or la protéine UCP1 dissipe ce gradient de protons. Il se produit alors une forte diminution de la production d'ATP et une augmentation de la production de chaleurs. Ainsi, pour une même production d'ATP, un adipocyte brun consommera beaucoup plus d'oxygène qu'un adipocyte blanc. La mesure de l'activité découplante est indiquée ci-après.

Dans la présente invention, l'expression d'un gène par une population cellulaire signifie que le produit issu de cette expression est soit le produit de traduction dudit gène, à savoir la protéine codée par le gène, soit le produit de transcription dudit gène, à savoir l'ARNm codant la protéine. L'expression d'un gène est déterminée par la quantification soit du produit de traduction (protéine codée par le gène), par exemple en utilisant la technique d'immunoempreinte, soit du produit de transcription (ARNm codant la protéine), par exemple par RT-PCR quantitative et/ou par la technique du Northern-blot. Les techniques de quantification des produits d'expression des gènes sont bien connues de l'homme de l'art.

L'expression des gènes codant les protéines UCP1, CIDEA, CPT1B, Bcl2, Bax, PPAR alpha, PGC1 alpha, PGC1 bêta et PRDM16 par la population d'adipocytes bruns humains fonctionnelle est quantifiée puis comparée à l'expression de ces mêmes gènes par une population d'adipocytes blancs humains. Cette population d'adipocytes blancs humains est avantageusement issue d'une population de cellules hMADS telle que décrite ci-dessus. Les conditions de culture permettant la différenciation des cellules hMADS en adipocytes blancs humains sont décrites notamment dans la demande internationale publiée le 12 février 2004 sous le numéro WO 2004/013275, page 17, ligne 36 à page 18, ligne 8, ou bien dans la partie Exemples de la présente demande de brevet. Avantageusement, la population d'adipocytes blancs humains est obtenue après stimulation d'une population cellulaire hMADS pendant 1 à 9 jours, avantageusement pendant 6 jours, par un agoniste spécifique du récepteur PPAR gamma tel qu'un composé de la famille des thiazolinediones, lequel composé est avantageusement choisi parmi la rosiglitazone, la ciglitazone, la pioglitazone, la darglitazone et la troglitazone.

Dans la présente invention, on considère que l'expression d'un gène par la population d'adipocytes bruns humains fonctionnelle est supérieure ou inférieure à celle du même gène par une population d'adipocytes blancs humains, lorsque l'on observe une différence significative de cette expression. A l'inverse, une différence n'est pas significative lorsque l'expression d'un gène par la population d'adipocytes bruns humains fonctionnelle est ± 0,20 fois identique à celle du même gène par une population d'adipocytes blancs humains.

Avantageusement, l'expression du gène codant la protéine UCP1 est 10 à 1000 fois supérieure, de préférence de 20 à 500 fois supérieure à l'expression de ce même gène par la population d'adipocytes blancs humains,
et/ou l'expression du gène codant la protéine CIDEA est 10 à 100 fois supérieure, de préférence 20 à 100 fois supérieure à l'expression de ce même gène par la population d'adipocytes blancs humains,
et/ou l'expression du gène codant la protéine CPT1B est 2 à 10 fois supérieure, de préférence 4 à 8 fois supérieure à l'expression de ce même gène par la population d'adipocytes blancs humains,
et/ou l'expression du gène codant la protéine Bcl2 est 1,5 à 4 fois supérieure, de préférence 2 à 3 fois supérieure à l'expression de ce même gène par la population d'adipocytes blancs humains,
et/ou l'expression du gène codant la protéine Bax est 0,25 à 2,5 fois inférieure, de préférence 0,5 à 2 fois inférieure à l'expression de ce même gène par la population d'adipocytes blancs humains,
et/ou l'expression des gènes codant les protéines PPAR alpha, PGC1 alpha, PGC1 bêta et PRDM16 est ± 0,20 fois identique à l'expression de ces mêmes gènes par la population d'adipocytes blancs humains.

L'expression du gène codant UCP1 est préférablement augmentée après stimulation par un agoniste d'un récepteur bêta-adrénergique, avantageusement après stimulation par un agoniste du récepteur bêta3-adrénergique, avantageusement pendant une durée comprise entre 1 à 24 heures, avantageusement 6 heures. Avantageusement, l'expression du gène codant UCP1 est 1,5 à 4 fois supérieure par rapport à celle obtenue en l'absence de stimulation par un agoniste d'un récepteur bêta-adrénergique.

Tout agoniste spécifique d'un récepteur bêta-adrénergique peut être utilisé. Des exemples de tels agonistes sont notamment cités dans le tableau 1 de Carpene, C. Methods Mol Biol 2001. 155, 129-140. On peut citer notamment, sans toutefois s'y limiter, les composés noradrénaline et adrénaline, les composés T0509 (beta1), les composés salbutamol et procaterol (beta2), le composé BRL37314 (beta3), la dobutamine, la terbutaline, l'isoprotérénol, qui est un agoniste spécifique des trois récepteurs bêta1-, bêta2- et bêta3-adrénergiques, le composé CGP12177A, qui est un agoniste partiel du récepteur bêta3-adrénergique, et le composé CL316243, qui est un agoniste spécifique du récepteur bêta3-adrénergique. Avantageusement, l'agoniste spécifique d'un récepteur bêta-adrénergique est choisi parmi la dobutamine, la terbutaline, l'isoprotérénol, la noradrénaline, l'adrénaline, le composé CGP12177A, qui est un agoniste partiel du récepteur bêta3-adrénergique, et le composé CL316243, qui est un agoniste spécifique du récepteur bêta3-adrénergique. Encore plus avantageusement, l'agoniste spécifique d'un récepteur bêta-adrénergique est choisi parmi les composés noradrénaline, adrénaline, isoprotérénol et CL316243. Les composés dobutamine, terbutaline isoprotérénol, et CL316243 peuvent notamment être obtenus auprès de Sigma Chemical Company (St Louis, USA). Le composé CGP12177A peut être obtenu auprès de Research Biochemical (Natick, MA, USA).

Egalement avantageusement, la concentration en agoniste spécifique d'un récepteur bêta-adrénergique est comprise entre 1 nM et 1000 nM, avantageusement entre 1 nM et 500 nM, encore plus avantageusement entre 1 et 100 nM.

L'invention décrit également l'utilisation d'un agoniste spécifique du récepteur bêta3-adrénergique pour augmenter l'expression du gène codant la protéine UCP1 dans une population d'adipocytes bruns humains telle que celle définie ici, avantageusement afin d'augmenter l'activité découplante de la protéine UCP1 dans ladite population. De préférence, l'agoniste est le composé CL316243, et est utilisé de manière préférée à une concentration comprise entre 1 nM et 1000 nM, avantageusement entre 1 nM et 500 nM, encore plus avantageusement entre 1 et 100 nM.

La présente invention a pour objet un procédé de différenciation d'une population de cellules souches humaines multipotentes dérivées du tissu adipeux (cellules hMADS) en une population d'adipocytes bruns humains fonctionnelle telle que définie ci-dessus, comprenant la stimulation desdites cellules hMADS par un agoniste spécifique du récepteur PPAR gamma, ledit agoniste étant une thiazolinedione, cette stimulation étant effectuée :
- soit pendant une période d'une durée comprise entre 10 et 30 jours, avantageusement entre 13 et 20 jours, encore plus avantageusement entre 15 et 16 jours,
- soit (i) pendant une première période de différenciation des cellules hMADS en adipocytes blancs d'une durée comprise entre 2 et 9 jours, avantageusement entre 3 et 7 jours, encore plus avantageusement de 6 jours, (ii) ladite première période étant suivie d'une seconde période d'une durée comprise entre 2 et 10 jours, avantageusement entre 4 et 7 jours, encore plus avantageusement de 5 jours, au cours de laquelle la stimulation est stoppée, (iii) ladite seconde période étant suivie d'une troisième période de stimulation d'une durée comprise entre 1 et 10 jours, avantageusement entre 1 et 6 jours, encore plus avantageusement de 2 jours.

Parmi les thiazolinediones pouvant être utilisées dans le cadre de la présente invention, on peut citer, sans toutefois s'y limiter, la rosiglitazone, la ciglitazone, la pioglitazone, la darglitazone, la troglitazone ou le composé NC-2100. Sont également décrits ici les agonistes spécifiques du récepteur PPAR gamma tels que le composé S26948, les composés dérivés de la N-(2-Benzoylphenyl)-L-tyrosine, les dérivés FMOC, les composés 1,3-dicarbonyle comportant des hétérocycles 2(3H)benzazolonique, les composés de la famille des sulfonylurées, les composés de la famille des acides 2-benzoylaminobenzoïques substitués en position 5, le composé halofénate, les dérivés de la famille des indene-N-oxydes, les antagonistes du récepteur de l'angiotensine de type 1 tels que le telmisartan, l'irbesartan et le losartan. Avantageusement, l'agoniste spécifique du récepteur PPAR gamma est une thiazolinedione, choisie dans le groupe constitué par la rosiglitazone, la ciglitazone, la pioglitazone, la darglitazone et la troglitazone. Les composés ciglitazone et troglitazone peuvent notamment être obtenus auprès de Cayman Chemical (Ann Arbor, MI, USA). Les composés pioglitazone et rosiglitazone peuvent être obtenus auprès de Sigma Chemical Co. (St Louis, MO, USA). La darglitazone peut être obtenue auprès de Medicinal Chemistry, AstraZeneca R&D (Molndal, Sweden).

Avantageusement, lorsque l'agoniste spécifique du récepteur PPAR gamma est la rosiglitazone, la concentration en rosiglitazone est comprise entre 5 nM et 1 000 nM, avantageusement entre 10 nM et 500 nM, encore plus avantageusement entre 20 et 100 nM. Egalement avantageusement, lorsque l'agoniste spécifique du récepteur PPAR gamma est la pioglitazone, la concentration en pioglitazone est comprise entre 0,2 µM et 10 µM, avantageusement entre 0,4 µM et 5 µM, encore plus avantageusement entre 0,8 µM et 2 µM. Encore avantageusement, lorsque l'agoniste spécifique du récepteur PPAR gamma est la ciglitazone, la concentration en ciglitazone est comprise entre 0,5 µM et 20 µM, avantageusement entre 1 µM et 10 µM, encore plus avantageusement entre 2 µM et 4 µM. Encore avantageusement, lorsque l'agoniste spécifique du récepteur PPAR gamma est la darglitazone, la concentration en darglitazone est comprise entre 0,2 µM et 20 µM, avantageusement entre 0,5 µM et 10 µM, encore plus avantageusement entre 1 µM et 5 µM. Encore avantageusement, lorsque l'agoniste spécifique du récepteur PPAR gamma est la troglitazone, la concentration en troglitazone est comprise entre 0,2 µM et 10 µM, avantageusement entre 0,5 µM et 5 µM, encore plus avantageusement entre 1 µM et 4 µM.

Les conditions générales de culture utilisées pour la multiplication et la différenciation de la population cellulaire hMADS sont connues de l'homme de l'art et sont notamment décrites dans la demande WO 2004/013275, ou bien dans la partie Exemples de la présente demande de brevet. Le milieu de culture utilisé pour la différenciation de la population cellulaire hMADS est changé régulièrement, de préférence tous les deux jours, avant utilisation des cellules. Ce milieu ne contient avantageusement pas de sérum tel que du sérum de veau nouveau-né et est chimiquement défini.

La fonctionnalité de la population d'adipocytes bruns humains selon l'invention peut en outre être vérifiée en stimulant son activité respiratoire à l'aide d'un agoniste spécifique d'un récepteur bêta-adrénergique tel que l'isoprotérénol. Ainsi, selon un mode de réalisation préféré, le procédé de différenciation de la population cellulaire hMADS en une population d'adipocytes bruns humains selon la présente invention, comprend en outre la vérification de la fonctionnalité de la population d'adipocytes bruns humains obtenue, ladite vérification comprenant les étapes successives suivantes :
- la stimulation de l'activité respiratoire de ladite population d'adipocytes bruns humains par un agoniste spécifique d'un récepteur bêta-adrénergique choisi dans le groupe constitué par l'isoprotérénol, la noradrénaline, l'adrénaline, la dobutamine, la terbutaline et le composé CL316243,
- la quantification de l'expression du gène codant UCP1 et/ou de la consommation d'oxygène, et
- la vérification que ladite population est fonctionnelle lorsque l'expression du gène codant UCP1 et/ou la consommation d'oxygène est augmentée par rapport à celle obtenue en l'absence de stimulation par ledit agoniste spécifique d'un récepteur bêta-adrénergique.

Lorsque la population est fonctionnelle, on observe une stimulation importante de l'activité respiratoire, telle qu'une augmentation d'au moins 20 %, de préférence au moins 40 % par rapport à l'absence de stimulation avec l'agoniste spécifique d'un récepteur bêta-adrénergique. En comparaison, aucune stimulation n'est observée lorsque les adipocytes blancs sont exposés à un agoniste spécifique d'un récepteur bêta-adrénergique.

De préférence, l'expression du gène codant UCP1 est 1,5 à 4 fois supérieure par rapport à celle obtenue en l'absence de stimulation par un agoniste d'un récepteur bêta-adrénergique.

Avantageusement, l'agoniste spécifique d'un récepteur bêta-adrénergique est l'isoprotérénol.

Egalement avantageusement, la concentration en agoniste spécifique d'un récepteur bêta-adrénergique est comprise entre 1 nM et 1000 nM, avantageusement entre 1 nM et 500 nM, encore plus avantageusement entre 1 et 100 nM.

La fonctionnalité de la population d'adipocytes bruns humains peut par exemple être vérifiée en quantifiant la consommation d'oxygène à l'aide d'un respiromètre. Une telle technique est bien connue de l'homme de l'art.

La fonctionnalité de la population d'adipocytes bruns humains peut également être vérifiée en quantifiant l'activité découplante de ladite population. Cette quantification de l'activité découplante peut être réalisée à l'aide d'un agent découplant tel que le carbonyl cyanide 3-chlorophénylhydrazone (CCCP). Une telle technique est bien connue de l'homme de l'art.

Selon un autre aspect, la présente invention a pour objet un procédé de conversion d'une population d'adipocytes blancs humains en une population d'adipocytes bruns humains fonctionnelle telle que définie dans la présente invention, comprenant la stimulation de ladite population d'adipocytes blancs humains par un agoniste spécifique du PPAR gamma, ledit agoniste étant une thiazolinedione, pendant une durée comprise entre 1 et 10 jours, avantageusement entre 1 et 6, encore plus avantageusement de 2 jours.

La population d'adipocytes blancs humains est telle que décrite ci-dessus. Le milieu de culture utilisé pour la conversion de la population d'adipocytes blancs humains est le même que celui utilisé pour la différenciation des cellules hMADS. De même, ce milieu est changé régulièrement, de préférence tous les deux jours, avant utilisation des cellules.

L'agoniste spécifique du récepteur PPAR gamma est tel que défini ci-dessus.

Avantageusement, ladite thiazolinedione est choisie dans le groupe constitué par la rosiglitazone, la ciglitazone, la pioglitazone, la darglitazone et la troglitazone.

Avantageusement, lorsque l'agoniste spécifique du récepteur PPAR gamma est la rosiglitazone, la concentration en rosiglitazone est comprise entre 5 nM et 1 000 nM, avantageusement entre 10 nM et 500 nM, encore plus avantageusement entre 20 et 100 nM. Egalement avantageusement, lorsque l'agoniste spécifique du récepteur PPAR gamma est la pioglitazone, la concentration en pioglitazone est comprise entre 0,2 µM et 10 µM, avantageusement entre 0,4 µM et 5 µM, encore plus avantageusement entre 0,8 µM et 2 µM. Encore avantageusement, lorsque l'agoniste spécifique du récepteur PPAR gamma est la ciglitazone, la concentration en ciglitazone est comprise entre 0,5 µM et 20 µM, avantageusement entre 1 µM et 10 µM, encore plus avantageusement entre 2 µM et 4 µM. Encore avantageusement, lorsque l'agoniste spécifique du récepteur PPAR gamma est la darglitazone, la concentration en darglitazone est comprise entre 0,2 µM et 20 µM, avantageusement entre 0,5 µM et 10 µM, encore plus avantageusement entre 1 µM et 5 µM. Encore avantageusement, lorsque l'agoniste spécifique du récepteur PPAR gamma est la troglitazone, la concentration en troglitazone est comprise entre 0,2 µM et 10 µM, avantageusement entre 0,5 µM et 5 µM, encore plus avantageusement entre 1 µM et 4 µM.

Dans un mode de réalisation préféré, le procédé de conversion selon la présente invention comprend en outre la vérification de la fonctionnalité de la population d'adipocytes bruns humains obtenue après conversion de la population d'adipocytes blancs humains, ladite vérification comprenant les étapes successives suivantes :
- la stimulation de l'activité respiratoire de ladite population d'adipocytes bruns humains par un agoniste spécifique d'un récepteur bêta-adrénergique choisi dans le groupe constitué par l'isoprotérénol, la noradrénaline, l'adrénaline, la dobutamine, la terbutaline et le composé CL316243,
- la quantification de l'expression du gène codant UCP1 et/ou de la consommation d'oxygène, et
- la vérification que ladite population est fonctionnelle lorsque l'expression du gène codant UCP1 et/ou la consommation d'oxygène est augmentée par rapport à celle obtenue en l'absence de stimulation par ledit agoniste spécifique d'un récepteur bêta-adrénergique.

De préférence, l'expression du gène codant UCP1 est 1,5 à 4 fois supérieure par rapport à celle obtenue en l'absence de stimulation par un agoniste d'un récepteur bêta-adrénergique.

Avantageusement, l'agoniste spécifique d'un récepteur bêta-adrénergique est l'isoprotérénol.

Encore avantageusement, la concentration en agoniste spécifique d'un récepteur bêta-adrénergique est comprise entre 1 nM et 1000 nM, avantageusement entre 1 nM et 500 nM, encore plus avantageusement entre 1 et 100 nM.

La présente invention décrit également la population d'adipocytes bruns humains obtenue par le procédé de différenciation ou le procédé de conversion tel que défini dans la présente invention.

Sont également décrits ici l'utilisation d'une population d'adipocytes bruns humains fonctionnelle telle que définie selon la présente invention en tant que modèle pour identifier une molécule ou une combinaison de molécules susceptible de moduler le poids corporel chez un sujet, avantageusement pour identifier une molécule ou une combinaison de molécules susceptible de traiter le surpoids et/ou l'obésité chez un sujet. Avantageusement, le sujet est un être humain.

Avantageusement, la présente invention décrit une méthode d'identification d'une molécule ou combinaison de molécules susceptible d'agir sur l'activité respiratoire et/ou l'activité découplante d'une population d'adipocytes bruns et/ou de moduler le poids corporel d'un sujet, comprenant les étapes successives suivantes :
- la mise en présence de la population d'adipocytes bruns humains fonctionnelle selon la présente invention avec ladite molécule ou combinaison de molécules,
- la quantification de l'expression du gène codant UCP1 et/ou de la consommation d'oxygène et/ou de l'activité découplante de ladite population d'adipocytes bruns humains, et
- l'identification de ladite molécule ou combinaison de molécules comme étant susceptible d'agir sur l'activité respiratoire et/ou l'activité découplante d'une population d'adipocytes bruns et/ou de moduler le poids corporel d'un sujet lorsque l'expression du gène codant UCP1 et/ou la consommation d'oxygène et/ou l'activité découplante est significativement différente de celle obtenue en l'absence de ladite molécule.

De manière avantageuse, lorsque l'expression du gène codant UCP1 et/ou la consommation d'oxygène et/ou l'activité découplante est supérieure à celle obtenue en l'absence de la molécule ou combinaison de molécules, ladite molécule ou combinaison de molécules est susceptible d'augmenter l'activité respiratoire et/ou l'activité découplante d'une population d'adipocytes bruns et/ou de traiter le surpoids et/ou l'obésité chez un sujet.

Egalement avantageusement, la présente invention décrit une méthode d'identification d'une molécule ou combinaison de molécules susceptible de favoriser la formation d'adipocytes bruns humains et/ou de traiter le surpoids et/ou l'obésité chez un sujet, comprenant les étapes successives suivantes :
a) la mise en oeuvre du procédé de différenciation ou de conversion, tel que décrit ci-dessus, de manière à obtenir une population de référence d'adipocytes bruns humains fonctionnelle,
b) la mise en présence d'une population de cellules hMADS avec ladite molécule ou combinaison de molécules,
c) l'identification de ladite molécule ou combinaison de molécules comme étant susceptible de favoriser la formation d'adipocytes bruns humains et/ou de traiter le surpoids et/ou l'obésité chez un sujet lorsque la population de cellules hMADS de l'étape b) exprime le phénotype de la population de référence d'adipocytes bruns humains fonctionnelle de l'étape a).

On entend par phénotype d'une population d'adipocytes bruns humains fonctionnelle selon la présente invention le fait que ladite population exprime simultanément les gènes codant les protéines UCP1, CIDEA, CPT1B, Bcl2, Bax, PPAR alpha, PGC1 alpha, PGC1 bêta et PRDM16 comme décrit plus haut. Egalement, ladite population possède une activité respiratoire élevée, qui peut être stimulée par un agoniste spécifique d'un récepteur bêta-adrénergique tel que l'isoprotérénol. Ce phénotype peut être déterminé comme décrit ci-dessus (immunoempreinte, Northern-blot, RT-PCR quantitative, respiromètre, etc...).

Encore avantageusement, la présente invention décrit une méthode d'identification d'une molécule ou combinaison de molécules susceptible de favoriser la formation d'adipocytes bruns humains et/ou de traiter le surpoids et/ou l'obésité chez un sujet, comprenant les étapes successives suivantes :
- la mise en présence d'une population d'adipocytes blancs humains avec ladite molécule ou combinaison de molécules,
- l'identification de ladite molécule ou combinaison de molécules comme étant susceptible de favoriser la formation d'adipocytes bruns humains et/ou de traiter le surpoids et/ou l'obésité chez un sujet lorsque la population d'adipocytes blancs humains exprime le phénotype d'une population d'adipocytes bruns humains telle que définie selon la présente invention.

La population d'adipocytes blancs humains est telle que définie ci-dessus.

Les exemples et figures qui suivent permettent d'illustrer la présente invention sans pour autant en limiter la portée.

### FIGURES

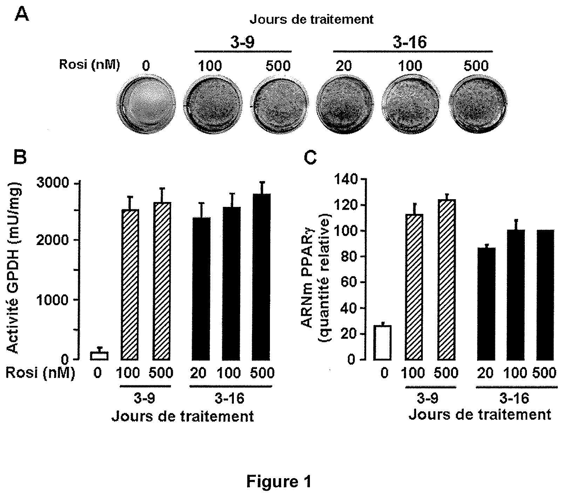

**Figure 1** **: Effet de la rosiglitazone sur la différenciation adipocytaire des cellules hMADS-2**
   La différenciation adipocytaire des cellules hMADS-2 a été menée selon le protocole décrit dans la partie « Matériels et Méthodes ». La rosiglitazone a été ajoutée aux concentrations et aux jours indiqués. Au jour 16 ont été réalisées, A) la fixation des cellules et leur coloration par l'huile rouge (Oil red O), B) la mesure de l'activité GPDH, et C) la détermination par RT-PCR quantitative des niveaux d'ARNm PPARγ. Les résultats représentent la moyenne ± SD de trois expériences indépendantes réalisées avec différentes séries de cellules ; 100% correspondent (C) à la valeur obtenue au jour 16 en présence de 500 nM rosiglitazone.

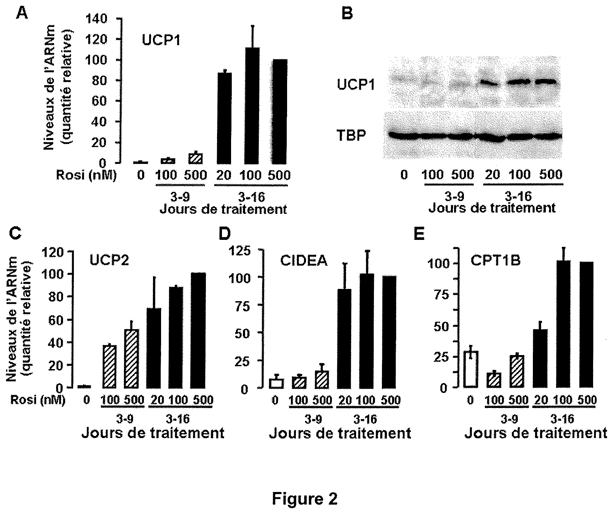

**Figure 2** **: Effet de l'exposition à long terme des cellules hMADS-2 à la rosiglitazone sur l'expression de marqueurs d'adipocytes bruns**
   La différenciation des cellules hMADS-2 a été réalisée comme décrit dans la Figure 1. Au jour 16, les niveaux d'ARNm UCP1 (A), UCP2 (C), CIDEA (D) et CPT1B (E) ont été déterminés par RT-PCR quantitative. Les résultats représentent la moyenne ±SD de trois expériences indépendantes réalisées avec différentes séries de cellules ; ils sont exprimés en prenant pour 100% la valeur obtenue en présence de 500 nM rosiglitazone. Les niveaux de la protéine UCP1 (B) ont été déterminés par immunoempreinte sur deux séries différentes de cellules en utilisant TBP comme standard interne endogène.

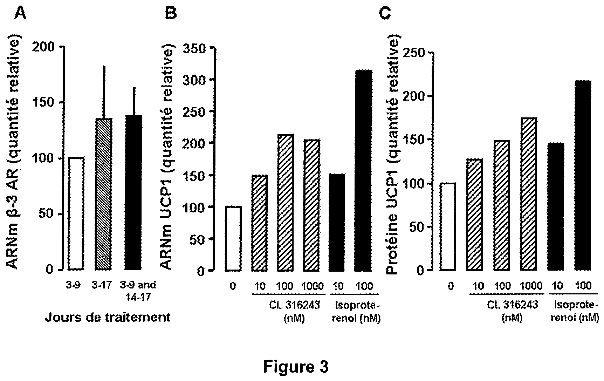

**Figure 3** **: Expression des récepteurs β3-adrénergiques et stimulation de l'expression d'UCP1 en réponse aux agonistes β-adrénergiques**
   A) la différenciation adipocytaire des cellules hMADS-2 a été réalisée en présence de 100nM rosiglitazone aux jours indiqués et les niveaux d'ARNm déterminés au jour 17 par RT-PCR quantitative. (B,C) la différenciation a été obtenue en présence de 100nM rosiglitazone entre les jours 3 et 16, en l'absence ou en la présence durant les 6 dernières heures d'agonistes β-adrénergiques aux concentrations indiquées. Les résultats représentent la moyenne ± SD de trois (A) et deux (B) expériences indépendantes réalisées avec différentes séries de cellules ; ils sont exprimés en prenant pour 100% les valeurs obtenues soit lors du traitement entre les jours 3 et 9 (A) soit en l'absence d'agonistes β-adrénergiques (B,C).

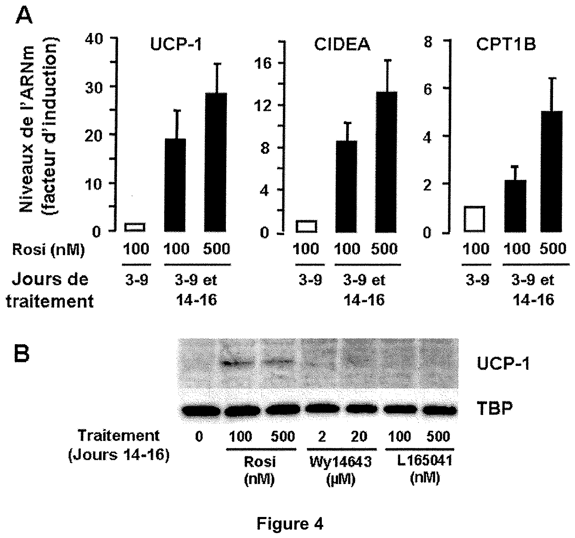

**Figure 4** **: L'induction d'un phénotype brun à partir d'adipocytes blancs dépend de l'activation de PPARγ**
   Dans un premier temps, les cellules hMADS-2 ont été différenciées en adipocytes blancs avec 100nM rosiglitazone présente entre les jours 3 et 9. Une fois éliminé, le ligand est rajouté ou pas au jour 14 pendant les jours suivants aux concentrations indiquées. Au jour 16, ont été déterminés les niveaux d'ARNm de l'UCP1, de CIDEA et de CPT1B par RT-PCR quantitative (A) et la quantité de protéine UCP1 a été déterminée par immunoempreinte après exposition entre les jours 14 et 16 aux ligands spécifiques des PPARs (B).
   Les résultats sont exprimés en facteur de stimulation en prenant pour 1 les valeurs obtenues au jour 16 après exposition à 100nM rosiglitazone entre les jours 3 et 9 ; les valeurs représentent les moyennes ± SD de deux expériences indépendantes réalisées avec différentes séries de cellules.

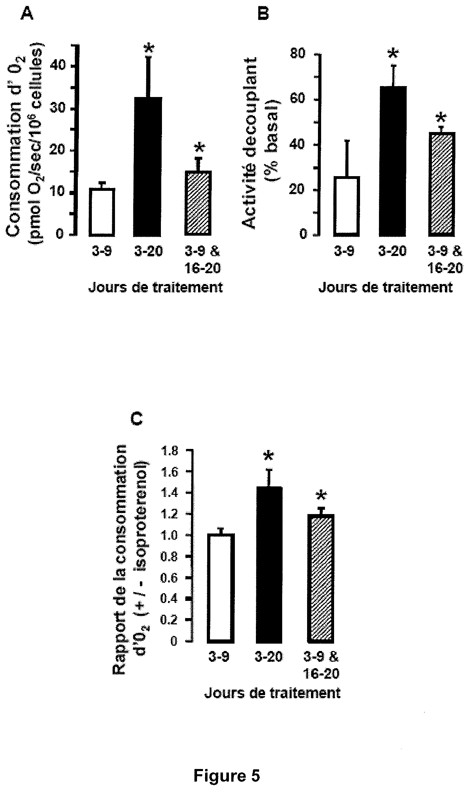

**Figure 5** **: Effet du traitement à long-terme par la rosiglitazone sur les activités respiratoire et découplante des adipocytes blancs et bruns**
   Les cellules hMADS-2 ont été induites à se différencier en présence de 100nM rosiglitazone soit pendant les jours 3 et 9 pour obtenir des adipocytes blancs, soit pendant les jours 3 et 20 pour obtenir des adipocytes bruns, soit enfin pendant les jours 3-9 suivi des jours 16-20. Au jour 20, la consommation d'oxygène (A), le découplage de la phosphorylation oxydative (B) et la stimulation de la consommation d'oxygène par l'isoprotérénol (C) ont été mesurés selon le protocole décrit dans « Matériels et Méthodes ». Les résultats représentent les moyennes ± SD de quatre expériences indépendantes réalisées avec différentes séries de cellules. *P<0,05 par comparaison avec les cellules traitées entre les jours 3 et 9 en présence de 100 nM rosiglitazone.

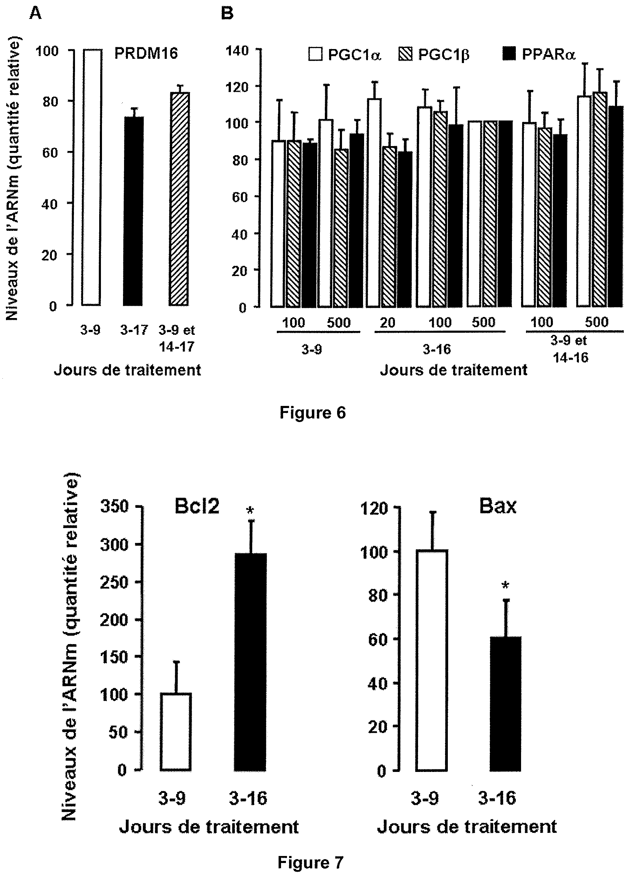

**Figure 6** **: Expression génique de facteurs et co-facteurs transcriptionnels en fonction de l'acquisition d'un phénotype blanc ou brun**
   La différenciation adipocytaire des cellules hMADS-2 a été réalisée en présence soit de rosiglitazone à 100nM (A) soit aux concentrations indiquées (B). Les niveaux d'ARNm PRDM16, PGC1α, PGC1β et PPARα ont été déterminés par RT-PCR quantitative au jour 16 (A) et au jour 17 (B). Les résultats représentent les moyennes ± SD de trois expériences indépendantes réalisées avec différentes séries de cellules ; ils sont exprimés en prenant pour 100% les valeurs obtenues en présence de 100nM (A) ou de 500nM rosiglitazone (B).
**Figure 7** **: Expression de gènes associés à l'apoptose en fonction de l'acquisition d'un phénotype blanc ou brun**
   La différenciation adipocytaire des cellules hMADS-2 a été réalisée en présence de 100nM rosiglitazone. Au jour 16, les niveaux d'ARNm Bcl2 et Bax ont été déterminés par RT-PCR quantitative. Les résultats représentent les moyennes ± SD de trois expériences indépendantes réalisées avec différentes séries de cellules ; ils sont exprimés en prenant pour 100% les valeurs obtenues lors du traitement par la rosiglitazone entre les jours 3 et 9. * P≤0,05.

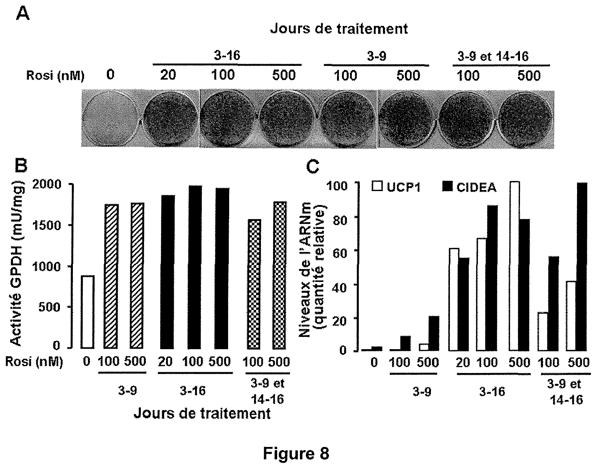

**Figure 8** **: Effet de la rosiglitazone sur la différenciation adipocytaire des cellules hMADS-1**
   Les cellules hMADS-1 ont été différenciées selon le protocole décrit dans la Figure 1. L'exposition à la rosiglitazone a été réalisée aux concentrations et aux jours indiqués. Au jour 16, les cellules ont été fixées et colorées à l'huile rouge (Oil Red O) (A), l'activité GPDH a été déterminée (B) ainsi que les niveaux d'ARNm UCP1 et CIDEA par RT-PCR quantitative (C). Les résultats représentent les moyennes ± SD de deux expériences indépendantes réalisées avec différentes séries de cellules et sont exprimés (C) en prenant pour 100% la valeur obtenue en présence de 100nM rosiglitazone au jour 16.

### EXEMPLES

Abréviations : cellules hMADS ("human multipotent adipose-derived stem cells"), cellules souches humaines multipotentes dérivées du tissu adipeux ; WAT (« white adipose tissue »), tissu adipeux blanc ; BAT (« brown adipose tissue »), tissu adipeux brun ; PPAR (« peroxisome proliferator-activated receptor »), récepteur activé par les inducteurs de la prolifération des peroxysomes ; PPRE (« peroxisome proliferator-responsive element »), élément de réponse des inducteurs de la prolifération des peroxysomes ; UCP1 (« uncoupling protein 1 »), protéine découplante 1 ; UCP2 (« uncoupling protein 2 »), protéine découplante 2 ; PGC-1α (β) (« PPARγ co-activator α(β) »), co-activateur α (β) du récepteur nucléaire PPAR gamma; CtBP-1 (« C-terminal-binding protein-1 »), protéine 1 de liaison C-terminale; AR (« adrenergic receptor »), récepteur adrénergique; CIDEA (« cell death-inducing DFF45-like effector A »), effecteur A de type DFF-45 induisant la mort cellulaire ; NAIP (« neuronal apoptosis inhibitory protein »), protéine d'inhibition de l'apoptose neuronale ; CTP-1B (« carnitine palmitoyltransferase-1B »), carnitine palmitoyltransférase-1B ; PKA (« protein kinase A »), protéine kinase A ; T3, 3,5,3'-tri-iodothyronine; TBP (« TATA-box binding protein »), protéine de liaison à la boîte TATA ; PRDM16 (« PR-domain zinc finger protein 16 »), protéine 16 contenant un domaine PR en doigt de zinc; Bax (« Bcl-2-associated X protein »), protéine X associée à Bcl-2; Bcl-2 (« B-cell CLL/lymphoma-2 »), lymphome-2/CLL à cellules B.

### I. Matériels et Méthodes

*Culture cellulaire* : L'établissement comme la caractérisation de la multipotence et de l'auto-renouvellement des cellules hMADS ont été décrits (Rodriguez, A.M., et al., Biochem Biophys Res Commun, 2004. 315(2): p. 255-63 ; Rodriguez, A.M., et al., J Exp Med, 2005. 201(9): p. 1397-405 ; Zaragosi, L.E. et al, Stem Cells, 2006. 24(11): p. 2412-9 ; Elabd, C., et al., Biochem Biophys Res Commun, 2007. 361(2): p. 342-8). Les cellules de la lignée hMADS-2 ont été établies à partir de tissu adipeux provenant de la région pubique d'un donneur âgé de 5 ans ; elles ont été utilisées entre les passages 16 à 35 (35 à 100 doublements de la population cellulaire). Les cellules ont été ensemencées à une densité de 4,500 cellules/cm² dans un milieu DMEM (Dulbecco's modified Eagle's medium) enrichi par 10% de sérum de veau fétal, 2,5ng /ml hFGF₂, 60µg/ml pénicilline et 50µg/ml streptomycine. Après un changement de milieu tous les deux jours, lorsque les cellules arrivent à confluence, hFGF₂ est éliminé et les cellules induites à se différencier deux jours après, définissant le jour 0 de différenciation. Le milieu de différenciation adipocytaire est constitué de DMEM/H12 (1/1 :v/v) enrichi en 10µg/ml transferrine, 0,85µM insuline, 0,2nM T₃, 1µM dexamethasone (DEX) et 500µM isobutylmethylxanthine (IBMX). Trois jours plus tard, le milieu est changé (DEX et IBMX omis) et la rosiglitazone ajoutée aux concentrations et aux jours indiqués. Le milieu est ensuite changé tous les deux jours avant utilisation des cellules. La détermination de l'activité glycérol-3 phosphate deshydrogénase (GPDH) et de la coloration des lipides par l'huile rouge (Oil Red O) ont été précédemment décrites (Negrel, R. et al, Proc Natl Acad Sci USA, 1978. 75(12): p. 6054-8 ; Bezy, O., et al., J Biol Chem, 2005. 280(12): p. 11432-8).

*Purification et analyse des ARNs* : L'extraction des ARNs, l'utilisation de la transcriptase inverse et la détermination des niveaux d'ARNm par RT-PCR quantitative en temps réel ont été décrites (Zaragosi, L.E. et al, Stem Cells, 2006. 24(11): p. 2412-9 ; Elabd, C., et al., Biochem Biophys Res Commun, 2007. 361(2): p. 342-8 ; Bezy, O., et al., J Biol Chem, 2005. 280(12): p. 11432-8). L'expression des gènes d'intérêt a été normalisée par rapport à celle du gène TBP et quantifiée à l'aide de la méthode comparative ΔCt. Les séquences des amorces oligonucléotidiques, obtenues à l'aide du Primer Express Software (Perkin Elmer Life Sciences), sont décrites dans le Tableau 1 ci-après.

**Tableau 1 : Séquence des amorces oligonucléotidiques pour l'analyse de l'expression génique**

| | **Amorce sens** | **Amorce anti-sens** | **N° d'accession** |
|---|---|---|---|
| FABP4 | TGTGCAGAAATGGGATGGAAA SEQ ID N° 1 | CAACGTCCCTTGGCTTATGCT SEQ ID N°2 | NM_001442 |
| UCP-1 | GTGTGCCCAACTGTGCAATG SEQ ID N°3 | CCAGGATCCAAGTCGCAAGA SEQ ID N°4 | NM_021833 |
| UCP-2 | GGCCTCACCGTGAGACCTTAC SEQ ID N°5 | TGGCCTTGAACCCAACCAT SEQ ID N°6 | NM_003355 |
| PPARγ | AGCCTCATGAAGAGCCTTCCA SEQ ID N°7 | TCCGGAAGAAACCCTTGCA SEQ ID N°8 | NM_005037 |
| PPARα | GGCGAACGATTCGACTCAAG SEQ ID N°9 | TCCAAAACGAATCGCGTTGT SEQ ID N°10 | NM_032644 |
| PGC-1α | CTGTGTCACCACCCAAATCCTTAT SEQ ID N°11 | TGTGTCGAGAAAAGGACCTTGA SEQ ID N°12 | NM_013261 |
| PGC-1b | GCGAGAAGTACGGCTTCATCAC SEQ ID N° 13 | CAGCGCCCTTTGTCAAAGAG SEQ ID N°14 | NM_133263 |
| PRDM16 | GAAACTTTATTGCCAATAGTGAGATGA SEQ ID N°15 | CCGTCCACGATCTGCATGT SEQ ID N°16 | NM_022114 |
| β3-AR | GCCTTCGCCTCCAACATG SEQ ID N°17 | AGCATCACGAGAAGAGGAAGGT SEQ ID N°18 | NM_000025 |
| CIDEA | GGCAGGTTCACGTGTGGATA SEQ ID N° 19 | GAAACACAGTGTTTGGCTCAAGA SEQ ID N°20 | NM_001279 |
| CPT1B | AAACAGTGCCAGGCGGTC SEQ ID N°21 | CGTCTGCCAACGCCTTG SEQ ID N°22 | NM_152246 |
| Bax | TGCCTCAGGATGCGTCCACCAA SEQ ID N°23 | CGGCAATCATCCTCTGCAGCTCCAT SEQ ID N°24 | NM_004324 |
| Bcl-2 | GCCCCCGTTGCTTTTCC SEQ ID N°25 | CCGGTTATCGTACCCTGTTCTC SEQ ID N°26 | NM_000657 |
| TBP | CACGAACCACGGCACTGATT SEQ ID N°27 | TTTTCTTGCTGCCAGTCTGGAC SEQ ID N°28 | NM_003194 |

*Analyse par immunoempreinte* : Les lysats cellulaires totaux sont analysés par immunoempreinte comme décrit précédemment (Bezy, O., et al., J Biol Chem, 2005. 280(12): p. 11432-8). Les anticorps primaires obtenus chez le lapin anti-UCP1 humain et anti-TBP sont des produits de Santa-Cruz (Sant-Cruz, USA) et les anticorps secondaires (conjugués à la peroxydase de raifort) des produits de Promega (Charbonnières, France). Le système « Enhanced ChemiLuminescence » (Millipore, Saint-Quentin-Yvelines, France) a été utilisé pour la détection.

*Détermination de la consommation d'oxygène* : La consommation d'oxygène a été mesurée à l'aide d'un respiromètre à 2 chambres d'injection équipé d'un thermostat Peltier, d'électrodes de type Clark et d'agitateurs magnétiques intégrés (Oxygraphe Oroboros, Insbrück, Autriche). Les mesures ont été faites à 37°C avec une agitation permanente dans un volume de 2ml de milieu DMEM/F12 (1/1 : v/v) contenant 10% de sérum de veau foetal. Avant chaque mesure, le milieu présent dans les chambres a été équilibré avec l'air pendant 30min, puis les cellules fraîchement trypsinées transférées dans ce milieu. Après avoir atteint un état respiratoire stationnaire, l'ATP synthase a été inhibée à l'aide d'oligomycine (0,25-0,5mg/l) et l'activité respiratoire des cellules titrée en présence de l'agent découplant carbonyl cyanide 3-chlorophénylhydrazone (CCCP) aux concentrations optimales de 1-2µM. La chaîne respiratoire a été bloquée par 1µg/ml d'Antimycine A. La consommation d'oxygène a été calculée à l'aide du logiciel DataGraph (Oroboros Software). L'activité respiratoire basale correspond à la consommation d'oxygène sensible à l'Antimycine A. La stimulation de l'activité respiratoire a été effectuée en présence de 1 µM isoprotérénol ajoutée extemporanément dans la chambre d'injection, les mesures étant faites comme décrite plus haut.

*Analyse statistique* : Les données sont exprimées en valeurs moyennes ± SD et sont analysées par le test t de Student. Les différences sont considérées comme significatives pour P ≤ 0,05.

### II. Résultats

### L'UCP1 et les marqueurs adipocytaires bruns sont exprimés au cours de la différenciation des cellules hMADS

Comme décrit précédemment (Rodriguez, A.M., et al., Biochem Biophys Res Commun, 2004. 315(2): p. 255-63), l'activation de PPARγ est nécessaire à la différenciation adipocytaire des cellules hMADS-2 (Fig.1A). Le traitement des cellules pendant 6 jours par des concentrations croissantes de rosiglitazone, entre les jours 3 et 9, conduit à une accumulation lipidique et à l'expression des gènes GPDH et PPARγ. Un traitement d'une semaine supplémentaire n'apporte pas de changements dans l'expression des gènes GPDH et PPARγ. Au jour 16, 20nM rosiglitazone suffisent à induire une réponse maximale, en accord avec l'affinité de PPARγ pour ce ligand (Fig.1A-C). L'ensemble des résultats souligne qu'une exposition de 6 jours des cellules hMADS-2 à la rosiglitazone permet l'expression maximale des marqueurs-clefs des adipocytes blancs. Par contre, une telle exposition entre les jours 3 et 9 ne conduit qu'à une très faible expression de l'ARNm et de la protéine UCP1. Toutefois une exposition à 20nM entre les jours 3 et 16 entraîne leur forte expression (Fig.2A,B). A l'inverse de l'UCP1, une forte expression de l'ARNm UCP2 est déjà observée au jour 9 ; elle est augmentée par une exposition plus longue (Fig.2C) et la protéine UCP2 est alors détectée (B. Miroux et C. Ricquier, communication personnelle). Ces résultats suggèrent que la durée du traitement par la rosiglitazone permet de moduler l'expression du gène UCP1. De même, l'expression du gène CIDEA, rapportée comme étroitement associée à celle de l'UCP1, est augmentée (Fig.2D) (Zhou, Z., et al., Nat Genet, 2003. 35(1): p. 49-56). Comparés aux adipocytes blancs, les adipocytes bruns présentent une mitochondriogénèse très importante (Wilson-Fritch, L., et al., J Clin Invest, 2004. 114(9): p. 1281-9). Effectivement, le contenu en ARNm codant pour la carnitine palmitoyltransférase mitochondriale (CPT1B) est fortement accru lorsque les cellules hMADS-2 basculent du phénotype blanc au phénotype brun (Fig.2E). Fait inattendu, les niveaux de PPARα, PGC-1α, PGC-1β et PRDM16 sont similaires chez les adipocytes exprimant les phénotypes blanc ou brun (Fig. 6). On sait que les adipocytes bruns de rongeurs sont plus susceptibles à l'apoptose que les adipocytes blancs *in vitro* comme *in vivo*. Ces adipocytes expriment à la fois la protéine anti-apoptotique Bcl-2 et la protéine pro-apoptotique Bax (Briscini et al., FEBS Lett 1998. 431, 80-84; Lindquist and Rehnmark, J Biol Chem1998. 273, 30147-30156; Nisoli et al., Cell Death Differ 2006. 13, 2154-2156). Contrairement au cas des rongeurs, les adipocytes blancs humains présentent une susceptibilité importante à l'apoptose qui paraît être liée à la faible expression des gènes anti-apoptotiques Bcl-2 et NAIP (Papineau et al., Metabolism 2003. 52, 987-992). Fait inattendu, le passage des cellules hMADS du phénotype blanc au phénotype brun s'accompagne d'une augmentation de l'expression du gène anti-apoptotique Bcl-2 et d'une diminution de celle du gène pro-apoptotique Bax, le rapport Bcl-2/Bax passant de 1 à 3,7 (Figure 7) impliquant, selon les espèces, un patron d'expression différent des gènes associés à l'apoptose. Dans la mesure où l'UCP1 (Fig.2), le récepteur β3-adrénergique (Fig.3A) et le récepteur β2-AR (résultat non montré) sont exprimés lorsque les cellules hMADS-2 sont exposées à la rosiglitazone entre les jours 3 et 16, la réponse fonctionnelle aux β-agonistes a été analysée. Comme l'indiquent les Figs.3B et 3C, l'expression de l'ARNm UCP1 et celle de la protéine UCP1 sont significativement augmentées après une stimulation pendant 6h par l'isoprotérénol, un pan-agoniste des récepteurs β, et par le composé CL316243, un agoniste β3 sélectif, pour des concentrations de 10 à 100nM. En résumé, une activation chronique prolongée de PPARγ entraîne l'expression d'UCP1 et l'acquisition d'une réponse fonctionnelle aux agonistes β.

### La régulation de l'expression d'UCP1 se produit dans les cellules hMADS préalablement différenciées en adipocytes blancs

Les expériences précédentes ne permettent pas de savoir si un traitement à long terme des cellules hMADS est nécessaire pour l'acquisition d'un phénotype brun, ou si une brève exposition à la rosiglitazone des cellules hMADS déjà différenciées en adipocytes blancs permet leur transdifférenciation. Dans ce but, les cellules hMADS-2 ont préalablement été exposées à la rosiglitazone entre les jours 3 et 9, le ligand éliminé puis rajouté entre les jours 14 et 16. Les résultats montrent que ce traitement de deux jours des adipocytes blancs suffit à stimuler l'expression des gènes UCP1, CIDEA et CPT1B (Fig.4A). Cet effet est spécifique à PPARγ, l'activation de PPARβ/δ et de PPARα par les ligands spécifiques Wy14643 et L165041, respectivement, n'induisant pas l'expression de la protéine UCP1. Le remplacement de la rosiglitazone par des acides gras polyinsaturés en tant qu'activateurs/ligands des PPARs (acides arachidonique, eicosapentaénoïque et docosahexaénoïque présents à 10µM) se révèle sans effet sur l'expression du gène UCP1 (résultats non montrés). L'ensemble de ces observations démontre qu'une activation spécifique de PPARγ pendant une brève période est suffisante pour que les adipocytes blancs acquièrent un phénotype d'adipocytes bruns. Les effets de la rosiglitazone sur l'expression d'UCP1 ne sont pas restreints aux cellules hMADS-2 ; ils sont également observés avec les cellules hMADS-1 et hMADS-3 (Rodriguez, A.M., et al., J Exp Med, 2005. 201(9): p. 1397-405), qui avaient été établies respectivement à partir du tissu adipeux de la région ombilicale d'une donneuse de 31 mois et de tissu adipeux pré-pubique d'un donneur de 4 mois (Fig. 8 et résultats non montrés).

### Consommation d'oxygène et découplage respiratoire des cellules hMADS différenciées en adipocytes blancs et bruns

Une caractéristique majeure des adipocytes bruns est une activité respiratoire intense et un découplage important de la phosphorylation oxydative. La consommation d'oxygène, déterminée à l'aide d'une électrode oxygéno-sensible (Cannon, B. et J. Nedergaard, Physiol Rev, 2004. 84(1): p. 277-359) a permis de mesurer les vitesses relatives de respiration. Les résultats montrent l'effet important d'un traitement à long-terme par la rosiglitazone sur les activités respiratoire totale et découplée. Après 20 jours d'exposition chronique qui permettent l'acquisition du phénotype brun, comparés aux valeurs obtenues avec les cellules hMADS-2 exposées entre les jours 3 et 9 et exprimant le phénotype blanc, ces deux activités sont augmentées de 3 fois et 2,5 fois, respectivement (Fig.5, A et B). Lorsque les cellules hMADS-2 sont préalablement différenciées en adipocytes blancs, puis traitées ultérieurement entre les jours 16 et 20 par la rosiglitazone, l'augmentation des activités respiratoire totale et découplée est moindre mais reste très notable (Fig. 5, A et B). Une stimulation importante de la consommation d'oxygène par un agoniste spécifique des récepteurs β-adénergique tel que l'isoprotérénol, est également observée lors de l'acquisition d'un phénotype brun (Fig. 5C). Ces résultats montrent que l'acquisition du phénotype brun par les cellules hMADS-2 s'accompagne comme attendu *via* UCP1 d'une augmentation de la consommation d'oxygène, de l'activité découplante et d'une stimulation de la respiration par un agoniste spécifique des récepteurs β-adrénergiques, démontrant que les adipocytes bruns obtenus à partir des cellules hMADS sont fonctionnels.

### III. Discussion

La technique par émission de positrons à l'aide du fluorodeoxyglucose a permis de démontrer récemment, chez l'homme adulte sain, la présence de tissu adipeux brun actif dans les sites distincts du tissu adipeux blanc (Nedergaard, J., T. et al, Am J Physiol Endocrinol Metab, 2007. 293(2): p. E444-52). Ainsi, contrairement au consensus qui a prévalu au cours des dernières décennies, ces observations importantes suggèrent la possibilité de stimuler l'activité métabolique du BAT afin de moduler les dépenses énergétiques chez l'homme. En effet, le tissu adipeux brun joue chez les rongeurs un rôle important dans la thermogénèse adaptative, son ablation par transgénèse conduisant à l'obésité et un dysfonctionnement étant observé chez les rongeurs obèses (Cannon, B. et J. Nedergaard, Physiol Rev, 2004. 84(1): p. 277-359 ; Lowell, B.B., et al., Nature, 1993. 366(6457): p. 740-2), alors que chez l'homme le rôle du BAT reste controversé (Cinti, S., Nutr Metab Cardiovasc Dis, 2006. 16(8): p. 569-74). Sur le plan pharmacologique, prenant en compte l'ensemble de ces observations, le développement d'un modèle d'adipocytes bruns humains devrait donc se révéler de première importance.

Nos résultats montrent pour la première fois que des cellules souches multipotentes humaines, établies à partir de tissu adipeux de jeunes donneurs et déjà connues pour se différencier en adipocytes blancs (Rodriguez, A.M., et al., Biochem Biophys Res Commun, 2004. 315(2): p. 255-63 ; Rodriguez, A.M., et al., J Exp Med, 2005. 201(9): p. 1397-405), sont également capables de donner naissance à des adipocytes bruns.

Sur le plan biologique, nos résultats permettent d'avancer l'hypothèse selon laquelle les cellules hMADS sont des cellules souches immatures dont le lignage se situerait en amont des lignages blanc et brun. Une fois engagées dans le lignage brun, les cellules hMADS présentent toutes les caractéristiques des adipocytes bruns de rongeurs; elles expriment les gènes UCP1, CIDEA, PGC-1α, PGC-1β et PRDM16 ainsi que les trois membres de la famille des PPARs. Fait crucial, l'acquisition du phénotype brun s'accompagne d'une augmentation importante des activités respiratoires et découplante. La modulation positive de l'expression d'UCP1 par l'isoprotérénol et par le composé CL316243 démontre que la voie de signalisation générée par les récepteurs β-adrénergiques, en particulier les récepteurs β3, est également fonctionnelle dans ces cellules.

Jusqu'à ce jour, la présence et le rôle des récepteurs β3-adrénergiques chez l'homme est restée controversée (Lafontan, M. et M. Berlan, Trends Pharmacol Sci, 2003. 24(6): p. 276-83). Ainsi les adipocytes bruns des jeunes babouins expriment faiblement ces récepteurs mais aucune lipolyse n'est observée en réponse à quatre agonistes β3-adrénergiques (Viguerie-Bascands, N., et al., J Clin Endocrinol Metab, 1996. 81(1): p. 368-75). Par ailleurs, des adipocytes bruns humains immortalisés par transgénèse et exprimant des récepteurs β3-adrénergiques ne montrent qu'une faible activité lipolytique en réponse au CGP12177A, un agoniste partiel β3, et ces récepteurs n'apparaissent que faiblement couplés à l'adénylate cyclase (Zilberfarb, V., et al., J Cell Sci, 1997. 110 (Pt 7): p. 801-7 ; Jockers, R., et al., Endocrinology, 1998. 139(6): p. 2676-84). Dans les deux cas, aucune stimulation de l'expression d'UCP1 et aucune activité respiratoire découplante n'avaient été rapportées en réponse à un agoniste spécifique β3, contrairement aux résultats de nos travaux. De plus, aucune stimulation de l'activité respiratoire par un agoniste spécifique des récepteurs β-adrénergiques n'avait été rapportée.

La rosiglitazone appartient à la famille des thiazolidinediones, une classe de molécules insulino-sensibilisantes utilisées dans le traitement du diabète de type 2 (Olefsky, J.M. et A.R. Saltiel, Trends Endocrinol Metab, 2000. 11(9): p. 362-8). Elle promeut la différenciation adipocytaire terminale en activant spécifiquement PPARγ (Rodriguez, A.M., et al., Biochem Biophys Res Commun, 2004. 315(2): p. 255-63 ; Tai, T.A., et al., J Biol Chem, 1996. 271(47): p. 29909-14 ; Forman, B.M., et al., Cell, 1995. 83(5): p. 803-12). L'activation de PPARγ se produit dans les préadipocytes blancs comme dans les préadipocytes bruns et conduit à leur différenciation en adipocytes blancs et bruns, respectivement (Nedergaard, J., et al., Biochim Biophys Acta, 2005. 1740(2): p. 293-304 ; Petrovic, N. et al., Am J Physiol Endocrinol Metab, (May 20, 2008). doi:10.1152/ajpendo.00035.2008).

Fait notable, malgré la présence de rosiglitazone et malgré le fait que l'activation de la voie PKA par le « cocktail » DEX/IBMX se révèle indispensable pendant les trois premiers jours de la différenciation, cet effet stimulateur se révèle insuffisant et seule une différenciation en adipocytes blancs se produit. Après élimination de DEX/IBMX du milieu de culture, il est frappant de constater que l'acquisition d'un phénotype d'adipocyte brun par les cellules hMADS ne dépend plus alors que de la durée d'activation de PPARγ par la rosiglitazone alors même que PGC-1α, PGC-1β et PRDM16 sont déjà pleinement exprimés dans les cellules exprimant le phénotype blanc.

On sait que chez la souris, PRDM16 induit dans les adipocytes blancs l'expression d'UCP1 cependant que l'activation de PPARγ est nécessaire à l'expression de CIDEA et des composants mitochondriaux (Seale, P., et al., Cell Metab, 2007. 6(1): p. 38-54). Nos résultats sont en accord avec ces observations et avec la présence d'un élément de réponse aux PPARs dans le promoteur du gène CIDEA (Viswakarma, N., et al., J Biol Chem, 2007. 282(25): p. 18613-24). Toutefois il n'est pas exclu que, outre l'expression de PRDM16, PGC-1α et PGC-1β, une exposition prolongée à la rosiglitazone n'induise d'autres événements moléculaires qui seraient également nécessaires à la pleine acquisition d'un phénotype brun. Une analyse transcriptomique différentielle entre cellules hMADS traitées brièvement ou longuement à la rosiglitazone devrait apporter des éléments de réponse à cette hypothèse.

La rosiglitazone, tout en normalisant la glycémie et l'insulinémie, entraîne une augmentation du poids corporel chez l'animal comme chez de nombreux patients (Carmona, M.C., et al., Int J Obes (Lond), 2005. 29(7): p. 864-71 ; Goldberg, R.B., Curr Opin Lipidol, 2007. 18(4): p. 435-42 ; Home, P.D., et al., Diabet Med, 2007. 24(6): p. 626-34 ; Joosen, A.M., et al., Diabetes Metab Res Rev, 2006. 22(3): p. 204-10). Nos résultats n'excluent pas la possibilité que, chez l'homme, elle puisse également -bien que de manière insuffisante- augmenter l'activité du BAT observé dans une proportion importante d'individus sains (Nedergaard, J. et al, Am J Physiol Endocrinol Metab, 2007. 293(2): p. E444-52 ; Cypess, AM et al, N. Engl. J. Med. 2009. 360 : p. 1509-17 ; Saito, M. et al, Diabetes 2009. Publish Ahead of Print, Online April 28; van Marken Lichtenbelt, W et al, N. Engl. J. Med. 2009. 390: p. 1500-08; Virtanen, KA et al, N. Engl. J. Med. 2009. 360 : p. 1518-1525).

La contribution du BAT aux dépenses énergétiques, dans le cas de la thermogénèse non-frissonante ou induite par un régime hypercalorique, est bien établie chez les rongeurs. Chez l'homme, les différences de gain de poids observées entre individus paraissent liées aux différences dans leur capacité à augmenter les dépenses énergétiques en réponse aux ingesta (Lowell, B.B. et E.S. Bachman, J Biol Chem, 2003. 278(32): p. 29385-8), et la masse de tissu adipeux brun est inversement proportionnelle à la masse du tissu adipeux blanc (Saito, M. et al, Diabetes 2009. Publish Ahead of Print, Online April 28; Virtanen, KA et al, N. Engl. J. Med. 2009. 360 : p. 1518-1525). Si ces observations sont liées à des capacités différentes entre individus à augmenter la masse et/ou l'activité du BAT, notre modèle d'adipocytes bruns humains devrait permettre le criblage de molécules capables d'augmenter la formation et les fonctions du BAT, en particulier en stimulant l'expression de PRDM16 et les capacités respiratoire et découplante des cellules. Parmi les possibilités, une augmentation de l'expression d'UCP1 pourrait être envisagée grâce à la double activation de la voie PKA *via* les récepteurs β-adrénergiques et *via* le récepteur TGR5 activé par les sels biliaires (Watanabe, M., et al., Nature, 2006. 439(7075): p. 484-9).

### IV. Résultats complémentaires

Les matériels et méthodes sont ceux indiqués au point I de la partie Exemples ci-dessus.
1- Des travaux récents ont montré chez la souris i) l'existence d'une signature myogénique des adipocytes bruns distincte de celle des adipocytes blancs (Timmons et al., 2007 ; Seale et al., 2008, Nature 454:961-967) et ii) la possibilité de générer des adipocytes bruns à partir de précurseurs blancs par traitement par la Bone Morphogentic Protein 7 (BMP7) (Tseng et al., 2008. Nature 454:1000-1004) ou par transgénèse (Tiraby, C et al, J. Biol ; Chem. 2003. 278 : p. 33370-76).
   Nous avons pu montrer que nos cellules humaines hMADS ne possèdent *pas* de signature musculaire puisqu'elles n'expriment pas le gène Myf5 ni pendant la phase de prolifération ni pendant et après leur différenciation en cellules adipeuses comme en cellules osseuses.
   De plus, le traitement par la seule BMP7 des cellules hMADS ne permet pas leur différenciation en adipocytes en l'absence de la rosiglitazone, mais entraîne par contre une faible augmentation de l'expression de la protéine UCP-1 sur des cellules préalablement différenciées en adipocytes blancs.
2- Les effets de la rosiglitazone sur la différenciation des cellules hMADS en adipocytes blancs et bruns sont médiés par le récepteur nucléaire PPARγ. En effet l'addition au milieu de différenciation d'un antagoniste de PPARγ, le composé GW 9662, empêche d'une part la différenciation des cellules hMADS en adipocytes, et ne permet pas d'autre part l'expression du gène UCP-1 dans des cellules préalablement différenciées en adipocytes blancs.
3- Comparés aux adipocytes blancs, les adipocytes bruns présentent une mitochondriogénèse très importante. Nous avions montré que le contenu en ARNm codant pour la carnitine palmitoyltransférase mitochondriale (CTP1B) est fortement accru lorsque les cellules hMADS-2 basculent du phénotype blanc au phénotype brun. Des résultats récents montrent que l'activité Cytochrome C oxydase (marqueur de la membrane interne mitochondriale) est également augmentée dans les adipocytes hMADS bruns comparé aux adipocytes blancs, renforçant ainsi nos observations sur l'augmentation de la mitochondriogénèse lors de la transition du phénotype blanc vers le phénotype brun.
4- Chez les rongeurs, les acides biliaires provenant de la réabsorption intestinale se lient à un récepteur couplé aux protéines G (TGR5) situé sur la membrane plasmique des adipocytes bruns. La production d'AMPc stimule l'expression d'une iodothyronine désiodinase de type 2 qui augmente les concentrations intracellulaires de T3. Celles-ci stimulent alors le découplage mitochondrial par l'intermédiaire de l'UCP et la dissipation d'énergie sous forme de chaleur (Watanabe et al., 2006). Chez l'homme, un tel système n'a jamais été décrit. Les cellules hMADS expriment le gène TGR5 au cours de la différenciation adipocytaire permettant ainsi d'envisager des études pharmacologiques à l'aide de ligands agonistes du récepteur TGR5 sur le découplage de la respiration.

### REFERENCES BIBLIOGRAPHIQUES

Ailhaud, G., Grimaldi, P., and Negrel, R. (1992). Cellular and molecular aspects of adipose tissue development. Annu Rev Nutr 12, 207-233.
Bezy, O., Elabd, C., Cochet, O., Petersen, R.K., Kristiansen, K., Dani, C., Ailhaud, G., and Amri, E.Z. (2005). Delta-interacting protein A, a new inhibitory partner of CCAAT/enhancer-binding protein beta, implicated in adipocyte differentiation. J Biol Chem 280, 11432-11438.
Bogacka, I., Xie, H., Bray, G.A., and Smith, S.R. (2005). Pioglitazone induces mitochondrial biogenesis in human subcutaneous adipose tissue in vivo. Diabetes 54, 1392-1399.
Briscini, L., Tonello, C., Dioni, L., Carruba, M.O., and Nisoli, E. (1998). Bcl-2 and Bax are involved in the sympathetic protection of brown adipocytes from obesity-linked apoptosis. FEBS Lett 431, 80-84.
Cannon, B., and Nedergaard, J. (2004). Brown adipose tissue: function and physiological significance. Physiol Rev 84, 277-359.
Carmona, M.C., Louche, K., Nibbelink, M., Prunet, B., Bross, A., Desbazeille, M., Dacquet, C., Renard, P., Casteilla, L., and Penicaud, L. (2005). Fenofibrate prevents Rosiglitazone-induced body weight gain in ob/ob mice. Int J Obes (Lond) 29, 864-871.
Casteilla, L., Champigny, O., Bouillaud, F., Robelin, J., and Ricquier, D. (1989). Sequential changes in the expression of mitochondrial protein mRNA during the development of brown adipose tissue in bovine and ovine species. Sudden occurrence of uncoupling protein mRNA during embryogenesis and its disappearance after birth. Biochem J 257, 665-671.
Casteilla, L., Forest, C., Robelin, J., Ricquier, D., Lombet, A., and Ailhaud, G. (1987). Characterization of mitochondrial-uncoupling protein in bovine fetus and newborn calf. Am J Physiol 252, E627-636.
Casteilla, L., Nougues, J., Reyne, Y., and Ricquier, D. (1991). Differentiation of ovine brown adipocyte precursor cells in a chemically defined serum-free medium. Importance of glucocorticoids and age of animals. Eur J Biochem 198, 195-199.
Cinti, S. (2006). The role of brown adipose tissue in human obesity. Nutr Metab Cardiovasc Dis 16, 569-574.
Cousin, B., Cinti, S., Morroni, M., Raimbault, S., Ricquier, D., Penicaud, L., and Casteilla, L. (1992). Occurrence of brown adipocytes in rat white adipose tissue: molecular and morphological characterization. J Cell Sci 103 (Pt 4), 931-942.
Cypess, A.M., Lehman, S., Williams, G., Tal, I., Rodman, D., Goldfine, A.B., Kuo, F.C., Palmer, E.L., Tseng, Y.H., Doria, A., Kolodny, G.M., and Kahn, C.R. (2009). Identification and importance of brown adipose tissue in adult humans. N Engl J Med 360, 1509-1517.
Elabd, C., Chiellini, C., Massoudi, A., Cochet, O., Zaragosi, L.E., Trojani, C., Michiels, J.F., Weiss, P., Carle, G., Rochet, N., et al. (2007). Human adipose tissue-derived multipotent stem cells differentiate in vitro and in vivo into osteocyte-like cells. Biochem Biophys Res Commun 361, 342-348.
Foellmi-Adams, L.A., Wyse, B.M., Herron, D., Nedergaard, J., and Kletzien, R.F. (1996). Induction of uncoupling protein in brown adipose tissue. Synergy between norepinephrine and pioglitazone, an insulin-sensitizing agent. Biochem Pharmacol 52, 693-701.
Forman, B.M., Tontonoz, P., Chen, J., Brun, R.P., Spiegelman, B.M., and Evans, R.M. (1995). 15-Deoxy-delta 12, 14-prostaglandin J2 is a ligand for the adipocyte determination factor PPAR gamma. Cell 83, 803-812.
Fukui, Y., Masui, S., Osada, S., Umesono, K., and Motojima, K. (2000). A new thiazolidinedione, NC-2100, which is a weak PPAR-gamma activator, exhibits potent antidiabetic effects and induces uncoupling protein 1 in white adipose tissue of KKAy obese mice. Diabetes 49, 759-767.
Garruti, G., and Ricquier, D. (1992). Analysis of uncoupling protein and its mRNA in adipose tissue deposits of adult humans. Int J Obes Relat Metab Disord 16, 383-390.
Goldberg, R.B. (2007). The new clinical trials with thiazolidinediones - DREAM, ADOPT, and CHICAGO: promises fulfilled? Curr Opin Lipidol 18, 435-442.
Home, P.D., Jones, N.P., Pocock, S.J., Beck-Nielsen, H., Gomis, R., Hanefeld, M., Komajda, M., and Curtis, P. (2007). Rosiglitazone RECORD study: glucose control outcomes at 18 months. Diabet Med 24, 626-634.
Jockers, R., Issad, T., Zilberfarb, V., de Coppet, P., Marullo, S., and Strosberg, A.D. (1998). Desensitization of the beta-adrenergic response in human brown adipocytes. Endocrinology 139, 2676-2684.
Joosen, A.M., Bakker, A.H., Gering, M.J., and Westerterp, K.R. (2006). The effect of the PPARgamma ligand rosiglitazone on energy balance regulation. Diabetes Metab Res Rev 22, 204-210.
Kelly, L.J., Vicario, P.P., Thompson, G.M., Candelore, M.R., Doebber, T.W., Ventre, J., Wu, M.S., Meurer, R., Forrest, M.J., Conner, M.W., et al. (1998). Peroxisome proliferator-activated receptors gamma and alpha mediate in vivo regulation of uncoupling protein (UCP-1, UCP-2, UCP-3) gene expression. Endocrinology 139, 4920-4927.
Lafontan, M., and Berlan, M. (2003). Do regional differences in adipocyte biology provide new pathophysiological insights? Trends Pharmacol Sci 24, 276-283.
Lindquist, J.M., and Rehnmark, S. (1998). Ambient temperature regulation of apoptosis in brown adipose tissue. Erk1/2 promotes norepinephrine-dependent cell survival. J Biol Chem 273, 30147-30156.
Lowell, B.B., and Bachman, E.S. (2003). Beta-Adrenergic receptors, diet-induced thermogenesis, and obesity. J Biol Chem 278, 29385-29388.
Lowell, B.B., V, S.S., Hamann, A., Lawitts, J.A., Himms-Hagen, J., Boyer, B.B., Kozak, L.P., and Flier, J.S. (1993). Development of obesity in transgenic mice after genetic ablation of brown adipose tissue. Nature 366, 740-742.
Mercer, S.W., and Trayhurn, P. (1986). Effects of ciglitazone on insulin resistance and thermogenic responsiveness to acute cold in brown adipose tissue of genetically obese (ob/ob) mice. FEBS Lett 195, 12-16.
Moulin, K., Truel, N., Andre, M., Arnauld, E., Nibbelink, M., Cousin, B., Dani, C., Penicaud, L., and Casteilla, L. (2001). Emergence during development of the white-adipocyte cell phenotype is independent of the brown-adipocyte cell phenotype. Biochem J 356, 659-664.
Nedergaard, J., Bengtsson, T., and Cannon, B. (2007). Unexpected evidence for active brown adipose tissue in adult humans. Am J Physiol Endocrinol Metab 293, E444-452.
Nedergaard, J., Petrovic, N., Lindgren, E.M., Jacobsson, A., and Cannon, B. (2005). PPARgamma in the control of brown adipocyte differentiation. Biochim Biophys Acta 1740, 293-304.
Negrel, R., Grimaldi, P., and Ailhaud, G. (1978). Establishment of preadipocyte clonal line from epididymal fat pad of ob/ob mouse that responds to insulin and to lipolytic hormones. Proc Natl Acad Sci U S A 75, 6054-6058.
Nisoli, E., Cardile, A., Bulbarelli, A., Tedesco, L., Bracale, R., Cozzi, V., Morroni, M., Cinti, S., Valerio, A., and Carruba, M.O. (2006). White adipocytes are less prone to apoptotic stimuli than brown adipocytes in rodent. Cell Death Differ 13, 2154-2156.
Olefsky, J.M., and Saltiel, A.R. (2000). PPAR gamma and the treatment of insulin resistance. Trends Endocrinol Metab 11, 362-368.
Papineau, D., Gagnon, A., and Sorisky, A. (2003). Apoptosis of human abdominal preadipocytes before and after differentiation into adipocytes in culture. Metabolism 52, 987-992.
Petrovic, N., Shabalina, I.G., Timmons, J.A., Cannon, B., and Nedergaard, J. (2008). Thermogenically Competent Non-Adrenergic Recruitment in Brown Predipocytes by a PPAR{gamma} Agonist. Am J Physiol Endocrinol Metab.
Puigserver, P., Rhee, J., Lin, J., Wu, Z., Yoon, J.C., Zhang, C.Y., Krauss, S., Mootha, V.K., Lowell, B.B., and Spiegelman, B.M. (2001). Cytokine stimulation of energy expenditure through p38 MAP kinase activation of PPARgamma coactivator-1. Mol Cell 8, 971-982.
Rodriguez, A.M., Elabd, C., Delteil, F., Astier, J., Vernochet, C., Saint-Marc, P., Guesnet, J., Guezennec, A., Amri, E.Z., Dani, C., et al. (2004). Adipocyte differentiation of multipotent cells established from human adipose tissue. Biochem Biophys Res Commun 315, 255-263.
Rodriguez, A.M., Pisani, D., Dechesne, C.A., Turc-Carel, C., Kurzenne, J.Y., Wdziekonski, B., Villageois, A., Bagnis, C., Breittmayer, J.P., Groux, H., et al. (2005). Transplantation of a multipotent cell population from human adipose tissue induces dystrophin expression in the immunocompetent mdx mouse. J Exp Med 201, 1397-1405.
Rosen, E.D., and Spiegelman, B.M. (2006). Adipocytes as regulators of energy balance and glucose homeostasis. Nature 444, 847-853.
Saito, M., Okamatsu-Ogura, Y., Matsushita, M., Watanabe, K., Yoneshiro, T., Nio-Kobayashi, J., Iwanaga, T., Miyagawa, M., Kameya, T., Nakada, K., Kawai, Y., and Tsujisaki, M. (2009). High Incidence of Metabolically Active Brown Adipose Tissue in Healthy Adult Humans: Effects of Cold Exposure and Adiposity. Diabetes*.*
Seale, P., Kajimura, S., Yang, W., Chin, S., Rohas, L.M., Uldry, M., Tavernier, G., Langin, D., and Spiegelman, B.M. (2007). Transcriptional Control of Brown Fat Determination by PRDM16. Cell Metab 6, 38-54.
Seale, P., Bjork, B., Yang, W., Kajimura, S., Chin, S., Kuang, S., Scime, A., Devarakonda, S., Conroe, H.M., Erdjument-Bromage, H., et al. 2008. PRDM16 controls a brown fat/skeletal muscle switch. Nature 454:961-967.
Tai, T.A., Jennermann, C., Brown, K.K., Oliver, B.B., MacGinnitie, M.A., Wilkison, W.O., Brown, H.R., Lehmann, J.M., Kliewer, S.A., Morris, D.C., et al. (1996). Activation of the nuclear receptor peroxisome proliferator-activated receptor gamma promotes brown adipocyte differentiation. J Biol Chem 271, 29909-29914.
Timmons, J.A., Wennmalm, K., Larsson, O., Walden, T.B., Lassmann, T., Petrovic, N., Hamilton, D.L., Gimeno, R.E., Wahlestedt, C., Baar, K., et al. (2007). Myogenic gene expression signature establishes that brown and white adipocytes originate from distinct cell lineages. Proc Natl Acad Sci U S A 104, 4401-4406.
Tiraby, C., and Langin, D. (2003). Conversion from white to brown adipocytes: a strategy for the control of fat mass? Trends Endocrinol Metab 14, 439-441.
Tiraby, C., Tavernier, G., Lefort, C., Larrouy, D., Bouillaud, F., Ricquier, D., and Langin, D. (2003). Acquirement of brown fat cell features by human white adipocytes. J Biol Chem 278, 33370-33376.
Tseng, Y.H., Kokkotou, E., Schulz, T.J., Huang, T.L., Winnay, J.N., Taniguchi, C.M., Tran, T.T., Suzuki, R., Espinoza, D.O., Yamamoto, Y., et al. 2008. New role of bone morphogenetic protein 7 in brown adipogenesis and energy expenditure. Nature 454:1000-1004.
Uldry, M., Yang, W., St-Pierre, J., Lin, J., Seale, P., and Spiegelman, B.M. (2006). Complementary action of the PGC-1 coactivators in mitochondrial biogenesis and brown fat differentiation. Cell Metab 3, 333-341.
van Marken Lichtenbelt, W.D., Vanhommerig, J.W., Smulders, N.M., Drossaerts, J.M., Kemerink, G.J., Bouvy, N.D., Schrauwen, P., and Teule, G.J. (2009). Cold-activated brown adipose tissue in healthy men. N Engl J Med 360, 1500-1508.
Viguerie-Bascands, N., Bousquet-Melou, A., Galitzky, J., Larrouy, D., Ricquier, D., Berlan, M., and Casteilla, L. (1996). Evidence for numerous brown adipocytes lacking functional beta 3-adrenoceptors in fat pads from nonhuman primates. J Clin Endocrinol Metab 81, 368-375.
Virtanen, K.A., Lidell, M.E., Orava, J., Heglind, M., Westergren, R., Niemi, T., Taittonen, M., Laine, J., Savisto, N.J., Enerback, S., and Nuutila, P. (2009). Functional brown adipose tissue in healthy adults. N Engl J Med 360, 1518-1525.
Viswakarma, N., Yu, S., Naik, S., Kashireddy, P., Matsumoto, K., Sarkar, J., Surapureddi, S., Jia, Y., Rao, M.S., and Reddy, J.K. (2007). Transcriptional regulation of Cidea, mitochondrial cell death-inducing DNA fragmentation factor alpha-like effector A, in mouse liver by peroxisome proliferator-activated receptor alpha and gamma. J Biol Chem 282, 18613-18624.
Watanabe, M., Houten, S.M., Mataki, C., Christoffolete, M.A., Kim, B.W., Sato, H., Messaddeq, N., Harney, J.W., Ezaki, O., Kodama, T., et al. (2006). Bile acids induce energy expenditure by promoting intracellular thyroid hormone activation. Nature 439, 484-489.
Wilson-Fritch, L., Nicoloro, S., Chouinard, M., Lazar, M.A., Chui, P.C., Leszyk, J., Straubhaar, J., Czech, M.P., and Corvera, S. (2004). Mitochondrial remodeling in adipose tissue associated with obesity and treatment with rosiglitazone. J Clin Invest 114, 1281-1289.
Xue, B., Coulter, A., Rim, J.S., Koza, R.A., and Kozak, L.P. (2005). Transcriptional synergy and the regulation of Ucp1 during brown adipocyte induction in white fat depots. Mol Cell Biol 25, 8311-8322.
Zaragosi, L.E., Ailhaud, G., and Dani, C. (2006). Autocrine fibroblast growth factor 2 signaling is critical for self-renewal of human multipotent adipose-derived stem cells. Stem Cells 24, 2412-2419.
Zhou, Z., Yon Toh, S., Chen, Z., Guo, K., Ng, C.P., Ponniah, S., Lin, S.C., Hong, W., and Li, P. (2003). Cidea-deficient mice have lean phenotype and are resistant to obesity. Nat Genet 35, 49-56.
Zilberfarb, V., Pietri-Rouxel, F., Jockers, R., Krief, S., Delouis, C., Issad, T., and Strosberg, A.D. (1997). Human immortalized brown adipocytes express functional beta3-adrenoceptor coupled to lipolysis. J Cell Sci 110 (Pt 7), 801-807.

## Revendications

1. Procédé de différenciation d'une population de cellules souches humaines multipotentes dérivées du tissu adipeux (cellules hMADS) en une population d'adipocytes bruns humains fonctionnelle, comprenant la stimulation desdites cellules hMADS par un agoniste spécifique du récepteur PPAR gamma, ledit agoniste étant une thiazolinedione, et cette stimulation étant effectuée :
- soit pendant une période d'une durée comprise entre 10 et 30 jours,
- soit (i) pendant une première période de différenciation des cellules hMADS en adipocytes blancs d'une durée comprise entre 2 et 9 jours, (ii) ladite première période étant suivie d'une seconde période d'une durée comprise entre 2 et 10 jours au cours de laquelle la stimulation est stoppée, (iii) ladite seconde période étant suivie d'une troisième période de stimulation d'une durée comprise entre 1 et 10 jours.

2. Procédé de différenciation selon la revendication 1, dans lequel ladite thiazolinedione est choisie dans le groupe constitué par la rosiglitazone, la ciglitazone, la pioglitazone, la darglitazone et la troglitazone.

3. Procédé de différenciation selon la revendication 2, dans lequel la concentration en agoniste spécifique du récepteur PPAR gamma est comprise entre 5 nM et 1000 nM lorsque l'agoniste est la rosiglitazone, entre 0,2 µM et 10 µM lorsque l'agoniste est la pioglitazone, entre 0,5 µM et 20 µM lorsque l'agoniste est la ciglitazone, entre 0,2 et 20 µM lorsque l'agoniste est la darglitazone et entre 0,2 et 10 µM lorsque l'agoniste est la troglitazone.

4. Procédé de différenciation selon l'une des revendications 1 à 3, comprenant en outre la vérification de la fonctionnalité de la population d'adipocytes bruns humains obtenue après différenciation de la population de cellules hMADS, ladite vérification comprenant les étapes successives suivantes :
- la stimulation de l'activité respiratoire de ladite population d'adipocytes bruns humains par un agoniste spécifique d'un récepteur bêta-adrénergique choisi dans le groupe constitué par l'isoprotérénol, la noradrénaline, l'adrénaline, la dobutamine, la terbutaline et le composé CL316243,
- la quantification de l'expression du gène codant UCP1 et/ou de la consommation d'oxygène, et
- la vérification que ladite population est fonctionnelle lorsque l'expression du gène codant UCP1 et/ou la consommation d'oxygène est augmentée par rapport à celle obtenue en l'absence de stimulation par ledit agoniste spécifique d'un récepteur bêta-adrénergique.

5. Procédé de différenciation selon la revendication 4, dans lequel l'agoniste spécifique d'un récepteur bêta-adrénergique est l'isoprotérénol.

6. Procédé de différenciation selon la revendication 4 ou 5, dans lequel la concentration en agoniste spécifique d'un récepteur bêta-adrénergique est comprise entre 1 nM et 1000 nM.

7. Procédé de conversion d'une population d'adipocytes blancs humains en une population d'adipocytes bruns humains fonctionnelle, comprenant la stimulation de ladite population d'adipocytes blancs humains par un agoniste spécifique du PPAR gamma pendant une durée comprise entre 1 et 10 jours, ledit agoniste étant une thiazolinedione.

8. Procédé de conversion selon la revendication 7, dans lequel ladite thiazolinedione est choisie dans le groupe constitué par la rosiglitazone, la ciglitazone, la pioglitazone, la darglitazone et la troglitazone.

9. Procédé de conversion selon la revendication 8, dans lequel la concentration en agoniste spécifique du récepteur PPAR gamma est comprise entre 5 nM et 1 000 nM lorsque l'agoniste est la rosiglitazone, entre 0,2 µM et 10 µM lorsque l'agoniste est la pioglitazone, entre 0,5 µM et 20 µM lorsque l'agoniste est la ciglitazone, entre 0,2 et 20 µM lorsque l'agoniste est la darglitazone et entre 0,2 et 10 µM lorsque l'agoniste est la troglitazone.

10. Procédé de conversion selon l'une des revendications 7 à 9, comprenant en outre la vérification de la fonctionnalité de la population d'adipocytes bruns humains obtenue après conversion de la population d'adipocytes blancs humains, ladite vérification comprenant les étapes successives suivantes :
- la stimulation de l'activité respiratoire de ladite population d'adipocytes bruns humains par un agoniste spécifique d'un récepteur bêta-adrénergique choisi dans le groupe constitué par l'isoprotérénol, la noradrénaline, l'adrénaline, la dobutamine, la terbutaline et le composé CL316243,
- la quantification de l'expression du gène codant UCP1 et/ou de la consommation d'oxygène, et
- la vérification que ladite population est fonctionnelle lorsque l'expression du gène codant UCP1 et/ou la consommation d'oxygène est augmentée par rapport à celle obtenue en l'absence de stimulation par ledit agoniste spécifique d'un récepteur bêta-adrénergique.

11. Procédé de conversion selon la revendication 10, dans lequel l'agoniste spécifique d'un récepteur bêta-adrénergique est l'isoprotérénol.

12. Procédé de conversion selon la revendication 10 ou 11, dans lequel la concentration en agoniste spécifique d'un récepteur bêta-adrénergique est comprise entre 1 nM et 1000 nM.

13. Méthode d'identification d'une molécule ou combinaison de molécules susceptible de favoriser la formation d'adipocytes bruns humains et/ou de traiter le surpoids et/ou l'obésité chez un sujet, comprenant les étapes successives suivantes :
a) la mise en oeuvre du procédé selon l'une quelconque des revendications 1 à 12, de manière à obtenir une population de référence d'adipocytes bruns humains fonctionnelle,
b) la mise en présence d'une population de cellules hMADS avec ladite molécule ou combinaison de molécules,
c) l'identification de ladite molécule ou combinaison de molécules comme étant susceptible de favoriser la formation d'adipocytes bruns humains et/ou de traiter le surpoids et/ou l'obésité chez un sujet lorsque la population de cellules hMADS de l'étape b) exprime le phénotype de la population de référence d'adipocytes bruns humains fonctionnelle de l'étape a).

## Patentansprüche

1. Differenzierungsverfahren einer aus adipösem Gewebe abgeleiteten, multipotenten, menschlichen Stammzellepopulation (hMADS-Zellen) in einer funktionale Population menschlicher brauner Adipozyten, umfassend die Stimulation der genannten hMADS-Zellen durch einen spezifischen Agonisten des Gamma-PPAR-Rezeptors, wobei der genannte Agonist ein Thiazolindion ist und diese Stimulation ausgeführt ist:
- entweder während einer Periode, die zwischen 10 und 30 Tagen umfassenden Dauer,
- oder (i) während einer ersten Differenzierungsperiode der hMADS-Zellen in weiße Adipozyten einer zwischen 2 und 9 Tagen umfassenden Dauer, (ii) wobei auf die erste Periode eine zweite Periode einer zwischen 2 und 10 Tagen umfassenden Dauer folgt, in deren Verlauf die Stimulation gestoppt ist, (iii) wobei auf die genannte zweite Periode eine dritte Stimulationsperiode einer zwischen 1 und 10 Tagen umfassenden Dauer folgt.

2. Differenzierungsverfahren gemäß Anspruch 1, wobei das genannte Thiazolindion aus der Gruppe ausgewählt ist, die aus Rosiglitazon, Ciglitazon, Pioglitazon, Darglitazon und Troglitazon besteht.

3. Differenzierungsverfahren gemäß Anspruch 2, wobei die Konzentration an spezifischem Agonisten des Gamma-PPAR-Rezeptors zwischen 5 nM und 1000 nM umfassend ist wenn der Agonist das Rosilitazon ist, zwischen 0,2 µM und 10 µM wenn der Agonist das Pioglitazon ist, zwischen 0,5 µM und 20 µM wenn der Agonist das Ciglitazon ist, zwischen 0,2 und 20 µM wenn der Agonist das Darglitazon ist, und zwischen 0,2 und 10 µM wenn der Agonist das Troglitazon ist.

4. Differenzierungsverfahren gemäß einem der Ansprüche 1 bis 3, umfassend darüber hinaus die Funktionalität der Population menschlicher brauner Adipozyten, die nach der Differenzierung der Population der hMADS-Zellen erhalten wurde, wobei die genannte Überprüfung die folgenden sukzessiven Schritte umfasst:
- die Stimulation der Atmungsaktivität der genannten Population menschlicher brauner Adipozyten durch einen spezifischen Agonisten eines adrenergischen Beta-Rezeptors, der ausgewählt ist aus der Gruppe, die aus Isoproterenol, Noradrenalin, Adrenalin, Dobutamin, Terbutalin und der Verbindung CL316243 besteht,
- die Quantifizierung der Expression des für UCP1-kodierenden Gens und / oder des Sauerstoffverbrauchs, und
- die Überprüfung, dass die genannte Population funktional ist, wenn die Expression des UCP1-kodierenden Gens und / oder des Sauerstoffverbrauchs im Verhältnis zu der erhöht ist, die bei Fehlen der Stimulation durch den genannten spezifischen Agonisten eines adrenergischen Beta-Rezeptors erhalten wird.

5. Differenzierungsverfahren gemäß Anspruch 4, wobei der spezifische Agonist eines adrenergischen Beta-Rezeptors das Isoproterol ist.

6. Differenzierungsverfahren gemäß Anspruch 4 oder 5, wobei die Konzentration an spezifischem Agonisten eines adrenergischen Beta-Rezeptoren zwischen 1 nM und 1000 nM umfassend ist.

7. Konvertierungsverfahren einer Population menschlicher weißer Adipozyten in eine funktionale Population menschlicher brauner Adipozyten, umfassend die Stimulation der genannten Population menschlicher weißer Adipozyten durch einen spezifischen Agonisten des Gamma-PPAR während einer zwischen 1 und 10 Tagen umfassenden Dauer, wobei der genannte Agonist ein Thiazolindion ist.

8. Konvertierungsverfahren gemäß Anspruch 7, wobei das genannte Thiazolindion ausgewählt ist aus der Gruppe, die aus Rosiglitazon, Ciglitazon, Pioglitazon, Darglitazon und Troglitazon besteht.

9. Konvertierungsverfahren gemäß Anspruch 8, wobei die Konzentration an spezifischem Agonisten des Gamma-PPAR-Rezeptors zwischen 5 nM und 1000 nM umfassend ist wenn der Agonist das Rosiglitazon ist, zwischen 0,2 µM und 10 wenn der Agonist das Pioglitazon ist, zwischen 0,5 µM und 20 µM wenn der Agonist das Ciglitazon ist, zwischen 0,2 und 20 µM wenn der Agonist das Darglitazon ist, und zwischen 0,2 und 10 µM wenn der Agonist das Troglitazon ist.

10. Konvertierungsverfahren gemäß einem der Ansprüche 7 bis 9, umfassend darüber hinaus die Überprüfung der Funktionalität der Population menschlicher brauner Adipozyten, die nach der Konvertierung der Population menschlicher weißer Adipozyten erhalten wird, wobei die genannte Überprüfung die folgenden sukzessiven Schritte umfasst:
- die Stimulation der Atmungsaktivität der genannten Population menschlicher brauner Adipozyten durch einen spezifischen Agonisten eines adrenergischen Beta-Rezeptors, der ausgewählt ist aus der Gruppe bestehend aus dem Isoproterenol, dem Noradrenalin, dem Adrenalin, dem Dobutamin, dem Terbutalin und der Verbindung CL316243,
- die Quantifizierung der Expression des UCP1-kodierenden Gens und /oder des Sauerstoffverbrauchs, und
- die Überprüfung, dass die genannte Population funktional ist, wenn die Expression des UCP1-kodierenden Gens und / oder der Sauerstoffverbrauch im Verhältnis zu der erhöht ist, die bei Fehlen der Stimulation durch den genannten spezifischen Agonisten eines adrenergischen Beta-Rezeptors erhalten wird.

11. Konvertierungsverfahren gemäß Anspruch 10, bei dem der spezifische Agonist eines adrenergischen Beta-Rezeptors das Isoproterenol ist.

12. Konvertierungsverfahren gemäß Anspruch 10 oder 11, bei dem die Konzentration an spezifischem Agonisten eines adrenergischen Beta-Rezeptors zwischen 1 nM und 1000 nM umfassend ist.

13. Identifizierungsverfahren eines Moleküls oder einer Molekülverbindung, das / die geeignet ist, die Bildung von menschlichen braunen Adipozyten zu begünstigen und / oder Übergewicht und / oder Fettsucht bei einem Patienten zu behandeln, umfassend die folgenden sukzessiven Schritte:
a) die Umsetzung des Verfahrens gemäß irgendeinem der Ansprüche 1 bis 12 derart, dass eine funktionale Referenzpopulation von menschlichen braunen Adipozyten erhalten wird,
b) das Gegenüberstellen einer Population von hMADS-Zellen mit dem genannten Molekül oder der genannten Molekülkombination,
c) die Identifizierung des genannten Moleküls oder der genannten Molekülkombination als geeignet zur Begünstigung der Bildung von menschlichen braunen Adipozyten und / oder zur Behandlung von Übergewicht und / oder Fettsucht bei einem Patienten, wenn die Population von hMADS-Zellen des Schritts b) den Phänotyp der funktionalen Referenzpopulation von menschlichen braunen Adipozyten des Schritts a) exprimiert.

## Claims

1. Method for differentiating a population of human multipotent adipose-derived stem cells (hMADS cells) into a functional population of human brown adipocytes, comprising the stimulation of said hMADS cells by a specific agonist of the gamma PPAR receptor, said agonist being a thiazolinedione, and this stimulation being carried out:
- either for a period of a duration comprised between 10 and 30 days,
- or (i) for a first period of differentiation of the hMADS cells into white adipocytes of a duration between 2 and 9 days, (ii) said first period being followed by a second period of a duration comprised between 2 and 10 days during which the stimulation is stopped, (iii) said second period being followed by a third period of stimulation of a duration comprised between 1 and 10 days.

2. Method for differentiating according to claim 1, wherein said thiazolinedione is chosen from the group consisting of rosiglitazone, ciglitazone, pioglitazone, darglitazone and troglitazone.

3. Method for differentiating according to claim 2, wherein the concentration in specific agonist of the gamma PPAR receptor is comprised between 5 nM and 1000 nM when the agonist is rosiglitazone, between 0.2 µM and 10 µM when the agonist is pioglitazone, between 0.5 µM and 20 µM when the agonist is ciglitazone, between 0.2 and 20 µM when the agonist is darglitazone and between 0.2 and 10 µM when the agonist is troglitazone.

4. Method for differentiating according to one of claims 1 to 3, further comprising the verification of the functionality of the population of human brown adipocytes obtained after differentiation of the hMADS cells population, said verification comprising the following successive steps:
- the stimulation of the respiratory activity of said population of human brown adipocytes by a specific agonist of a beta-adrenergic receptor chosen from the group consisting of isoproterenol, noradrenaline, adrenaline, dobutamine, terbutaline and the compound CL316243,
- the quantification of the expression of the gene encoding UCP1 and/or of the oxygen consumption, and
- the verification that said population is functional when the expression of the gene encoding UCP1 and/or the oxygen consumption is increased with respect to that obtained in the absence of stimulation by said specific agonist of a beta-adrenergic receptor.

5. Method for differentiating according to claim 4, wherein the specific agonist of a beta-adrenergic receptor is isoproterenol.

6. Method for differentiating according to claim 4 or 5, wherein the concentration in specific agonist of a beta-adrenergic receptor is comprised between 1 nM and 1000 nM.

7. Method for the conversion of a population of white human adipocytes into a functional population of human brown adipocytes, comprising the stimulation of said population of white human adipocytes with a specific agonist of the PPAR gamma for a duration comprised between 1 and 10 days, said agonist being a thiazolinedione.

8. Method for the conversion according to claim 7, wherein said thiazolinedione is chosen from the group consisting of rosiglitazone, ciglitazone, pioglitazone, darglitazone and troglitazone.

9. Method for the conversion according to claim 8, wherein the concentration in specific agonist of the gamma PPAR receptor is between 5 nM and 1000 nM when the agonist is rosiglitazone, between 0.2 µM and 10 µM when the agonist is pioglitazone, between 0.5 µM and 20 µM when the agonist is ciglitazone, between 0.2 and 20 µM when the agonist is darglitazone and between 0.2 and 10 µM when the agonist is troglitazone.

10. Method for the conversion according to one of claims 7 to 9, further comprising the verification of the functionality of the population of human brown adipocytes obtained after conversion of the population of white human adipocytes, said verification comprising the following successive steps:
- the stimulation of the respiratory activity of said population of human brown adipocytes by a specific agonist of a beta-adrenergic receptor chosen from the group consisting of isoproterenol, noradrenaline, adrenaline, dobutamine, terbutaline and the compound CL316243,
- the quantification of the expression of the gene encoding UCP1 and/or of the oxygen consumption, and
- the verification that said population is functional when the expression of the gene encoding UCP1 and/or the oxygen consumption is increased with respect to that obtained in the absence of stimulation by said specific agonist of a beta-adrenergic receptor.

11. Method for the conversion according to claim 10, wherein the specific agonist of a beta-adrenergic receptor is isoproterenol.

12. Method for the conversion according to claim 10 or 11, wherein the concentration in specific agonist of a beta-adrenergic receptor is comprised between 1 nM and 1000 nM.

13. Method for identifying a molecule or combination of molecules capable of favouring the formation of human brown adipocytes and/or to treat excess weight and/or obesity in a subject, comprising the following successive steps:
a) implementing the method according to any of claims 1 to 12, so as to obtain a population of reference of functional human brown adipocytes,
b) contacting a population of hMADS cells with said molecule or combination of molecules,
c) identifying said molecule or combination of molecules as being capable of favouring the formation of human brown adipocytes and/or of treating excess weight and/or obesity in a subject when the hMADS cells population of step b) expresses the phenotype of the population of reference of functional human brown adipocytes of step a).


### SEQUENCE LISTING

<110> CENTRE NATIONAL DE LA RECHERCHE SCIENTIFIQUE (CNRS) AILHAUD, Gérard AMRI, Ez-Zoubir DANI, Christian ELABD, Christian
<120> Lignée établie d'adipocytes bruns humains et procédé de différenciation à partir d'une lignée de cellules hMADS
<130> 354865
<150> FR0854140
   <151> 2008-06-23
<160> 28
<170> PatentIn version 3.3
<210> 1
   <211> 21
   <212> ADN
   <213> Séquence artificielle
<220>
   <223> amorce sens FABP4
<400> 1
   tgtgcagaaa tgggatggaa a 21
<210> 2
   <211> 21
   <212> ADN
   <213> Séquence artificielle
<220>
   <223> amorce anti-sens FABP4
<400> 2
   caacgtccct tggcttatgc t 21
<210> 3
   <211> 20
   <212> ADN
   <213> Séquence artificielle
<220>
   <223> amorce sens UCP-1
<400> 3
   gtgtgcccaa ctgtgcaatg 20
<210> 4
   <211> 20
   <212> ADN
   <213> Séquence artificielle
<220>
   <223> amorce anti-sens UCP-1
<400> 4
   ccaggatcca agtcgcaaga 20
<210> 5
   <211> 21
   <212> ADN
   <213> Séquence artificielle
<220>
   <223> amorce sens UCP-2
<400> 5
   ggcctcaccg tgagacctta c 21
<210> 6
   <211> 19
   <212> ADN
   <213> Séquence artificielle
<220>
   <223> amorce anti-sens UCP-2
<400> 6
   tggccttgaa cccaaccat 19
<210> 7
   <211> 21
   <212> ADN
   <213> Séquence artificielle
<220>
   <223> amorce sens PPAR gamma
<400> 7
   agcctcatga agagccttcc a 21
<210> 8
   <211> 19
   <212> ADN
   <213> Séquence artificielle
<220>
   <223> amorce anti-sens PPAR gamma
<400> 8
   tccggaagaa acccttgca 19
<210> 9
   <211> 20
   <212> ADN
   <213> Séquence artificielle
<220>
   <223> amorce sens PPAR alpha
<400> 9
   ggcgaacgat tcgactcaag 20
<210> 10
   <211> 20
   <212> ADN
   <213> Séquence artificielle
<220>
   <223> amorce anti-sens PPAR alpha
<400> 10
   tccaaaacga atcgcgttgt 20
<210> 11
   <211> 24
   <212> ADN
   <213> Séquence artificielle
<220>
   <223> amorce sens PGC-1 alpha
<400> 11
   ctgtgtcacc acccaaatcc ttat 24
<210> 12
   <211> 22
   <212> ADN
   <213> Séquence artificielle
<220>
   <223> amorce anti-sens PGC-1 alpha
<400> 12
   tgtgtcgaga aaaggacctt ga 22
<210> 13
   <211> 22
   <212> ADN
   <213> Séquence artificielle
<220>
   <223> amorce sens PGC-1 beta
<400> 13
   gcgagaagta cggcttcatc ac 22
<210> 14
   <211> 20
   <212> ADN
   <213> Séquence artificielle
<220>
   <223> amorce anti-sens PGC-1 beta
<400> 14
   cagcgccctt tgtcaaagag 20
<210> 15
   <211> 27
   <212> ADN
   <213> Séquence artificielle
<220>
   <223> amorce sens PRDM16
<400> 15
   gaaactttat tgccaatagt gagatga 27
<210> 16
   <211> 19
   <212> ADN
   <213> Séquence artificielle
<220>
   <223> amorce anti-sens PRDM16
<400> 16
   ccgtccacga tctgcatgt 19
<210> 17
   <211> 18
   <212> ADN
   <213> Séquence artificielle
<220>
   <223> amorce sens beta 3-AR
<400> 17
   gccttcgcct ccaacatg 18
<210> 18
   <211> 22
   <212> ADN
   <213> Séquence artificielle
<220>
   <223> amorce anti-sens beta 3-AR
<400> 18
   agcatcacga gaagaggaag gt 22
<210> 19
   <211> 20
   <212> ADN
   <213> Séquence artificielle
<220>
   <223> amorce sens CIDEA
<400> 19
   ggcaggttca cgtgtggata 20
<210> 20
   <211> 23
   <212> ADN
   <213> Séquence artificielle
<220>
   <223> amorce anti-sens CIDEA
<400> 20
   gaaacacagt gtttggctca aga 23
<210> 21
   <211> 18
   <212> ADN
   <213> Séquence artificielle
<220>
   <223> amorce sens CPT1B
<400> 21
   aaacagtgcc aggcggtc 18
<210> 22
   <211> 17
   <212> ADN
   <213> Séquence artificielle
<220>
   <223> amorce anti-sens CPT1B
<400> 22
   cgtctgccaa cgccttg 17
<210> 23
   <211> 22
   <212> ADN
   <213> Séquence artificielle
<220>
   <223> amorce sens Bax
<400> 23
   tgcctcagga tgcgtccacc aa 22
<210> 24
   <211> 25
   <212> ADN
   <213> Séquence artificielle
<220>
   <223> amorce anti-sens Bax
<400> 24
   cggcaatcat cctctgcagc tccat 25
<210> 25
   <211> 17
   <212> ADN
   <213> Séquence artificielle
<220>
   <223> amorce sens Bcl-2
<400> 25
   gcccccgttg cttttcc 17
<210> 26
   <211> 22
   <212> ADN
   <213> Séquence artificielle
<220>
   <223> amorce anti-sens Bcl-2
<400> 26
   ccggttatcg taccctgttc tc 22
<210> 27
   <211> 20
   <212> ADN
   <213> Séquence artificielle
<220>
   <223> amorce sens TBP
<400> 27
   cacgaaccac ggcactgatt 20
<210> 28
   <211> 22
   <212> ADN
   <213> Séquence artificielle
<220>
   <223> amorce anti-sens TBP
<400> 28
   ttttcttgct gccagtctgg ac 22